# EUROPEAN PATENT SPECIFICATION

(11) **EP 2 380 972 B1**
(45) Date of publication and mention of the grant of the patent: **19.09.2012**
(21) Application number: 10160288.6
(22) Date of filing: 19.04.2010
(51) Int. Cl.: C12N 5/0797

(54) **Methods and compositions for the expansion of somatic stem cells and progenitor cells**
Verfahren und Zusammensetzungen zur Ausdehnung von somatischen Stammzellen und Vorläuferzellen
Procédés et compositions pour l'expansion de cellules souches somatiques et de cellules progénitrices

(43) Date of publication of application: 26.10.2011
(73) Proprietor: Technische Universität Dresden, 01069 Dresden (DE)
(72) Inventor: Calegari, Federico, Dr., 01326 Dresden (DE); Artegiani, Benedetta, 01277 Dresden (DE); Waskow, Claudia, Dr., 01309 Dresden (DE); Grinenko, Tatyana, Dr., 01097 Dresden (DE)
(74) Representative: Kailuweit & Uhlemann Patentanwälte

(56) References cited:
- WO-A2-2008/137122
- ZHAO CHUNNIAN ET AL: "MicroRNA let-7b regulates neural stem cell proliferation and differentiation by targeting nuclear receptor TLX signaling" PROCEEDINGS OF THE NATIONAL ACADEMY OF SCIENCES OF THE UNITED STATES OF AMERICA, vol. 107, no. 5, February 2010 (2010-02), pages 1876-1881, XP9134658 ISSN: 0027-8424
- LANGE CHRISTIAN ET AL: "Cdk4/cyclinD1 overexpression in neural stem cells shortens G1, delays neurogenesis, and promotes the generation and expansion of basal progenitors." CELL STEM CELL 4 SEP 2009 LNKD- PUBMED:19733543, vol. 5, no. 3, 4 September 2009 (2009-09-04), pages 320-331, XP9134639 ISSN: 1875-9777
- BRYJA V ET AL: "Lineage specific composition of cyclin D-CDK4/CDK6-p27 complexes reveals distinct functions of CDK4, CDK6 and individual D-type cyclins in differentiating cells of embryonic origin" CELL PROLIFERATION, vol. 41, no. 6, December 2008 (2008-12), pages 875-893, XP002587273 ISSN: 0960-7722
- SALOMONI PAOLO ET AL: "Cell cycle control of mammalian neural stem cells: putting a speed limit on G1." TRENDS IN CELL BIOLOGY MAY 2010 LNKD- PUBMED:20153966, vol. 20, no. 5, 12 February 2010 (2010-02-12), pages 233-243, XP002587274 ISSN: 1879-3088 [retrieved on 2010-02-12]

### SEQUENCE LISTING

<110> Technische Universität Dresden
<120> METHODS AND COMPOSITIONS FOR THE EXPANSION OF SOMATIC STEM CELLS AND PROGENITOR CELLS
<130> 00017P0153EP
<160> 45
<170> PatentIn version 3.3
<210> 1
   <211> 16
   <212> PRT
   <213> Artificial
<220>
   <223> Peptide
<400> 1
   Arg Gln Ile Lys Ile Trp Phe Gln Asn Arg Arg Met Lys Trp Lys Lys
   1               5                   10                  15
<210> 2
   <211> 18
   <212> PRT
   <213> Artificial
<220>
   <223> Peptide
<400> 2 Leu Ala
<210> 3
   <211> 9
   <212> PRT
   <213> Artificial
<220>
   <223> Peptide
<400> 3
   Arg Lys Lys Arg Arg Gln Arg Arg Arg
   1 5
<210> 4
   <211> 5
   <212> PRT
   <213> Artificial
<220>
   <223> Peptide
<220>
   <221> misc_feature
   <222> (2)..(2)
   <223> Xaa can be any naturally occurring amino acid
<400> 4
   Glu Xaa Asn Pro Gly
   1 5
<210> 5
   <211> 7
   <212> PRT
   <213> Artificial
<220>
   <223> Peptide
<220>
   <221> misc_feature
   <222> (2)..(2)
   <223> Xaa can be any naturally occurring amino acid
<220>
   <221> misc_feature
   <222> (4)..(4)
   <223> Xaa can be any naturally occurring amino acid
<400> 5
   Asp Xaa Glu Xaa Asn Pro Gly
   1 5
<210> 6
   <211> 7
   <212> PRT
   <213> Artificial
<220>
   <223> Peptide
<220>
   <221> misc_feature
   <222> (2)..(2)
   <223> Xaa can be any naturally occurring amino acid
<220>
   <221> misc_feature
   <222> (4)..(4)
   <223> Xaa can be any naturally occurring amino acid
<400> 6
   Gly Xaa Glu Xaa Asn Pro Gly
   1 5
<210> 7
   <211> 7
   <212> PRT
   <213> Artificial
<220>
   <223> Peptide
<220>
   <221> misc_feature
   <222> (4)..(4)
   <223> xaa can be any naturally occurring amino acid
<400> 7
   Asp Val Glu Xaa Asn Pro Gly
   1 5
<210> 8
   <211> 7
   <212> PRT
   <213> Artificial
<220>
   <223> Peptide
<220>
   <221> misc_feature
   <222> (4)..(4)
   <223> xaa can be any naturally occurring amino acid
<400> 8
   Asp Ile Glu Xaa Asn Pro Gly
   1               5
<210> 9
   <211> 7
   <212> PRT
   <213> Artificial
<220>
   <223> Peptide
<220>
   <221> misc_feature
   <222> (4)..(4)
   <223> Xaa can be any naturally occurring amino acid
<400> 9
   Asp Leu Glu Xaa Asn Pro Gly
   1               5
<210> 10
   <211> 7
   <212> PRT
   <213> Artificial
<220>
   <223> Peptide
<220>
   <221> misc_feature
   <222> (4)..(4)
   <223> Xaa can be any naturally occurring amino acid
<400> 10
   Gly Val Glu Xaa Asn Pro Gly
   1               5
<210> 11
   <211> 7
   <212> PRT
   <213> Artificial
<220>
   <223> Peptide
<220>
   <221> misc_feature
   <222> (4)..(4)
   <223> Xaa can be any naturally occurring amino acid
<400> 11
   Gly Ile Glu Xaa Asn Pro Gly
   1               5
<210> 12
   <211> 7
   <212> PRT
   <213> Artificial
<220>
   <223> Peptide
<220>
   <221> misc_feature
   <222> (4)..(4)
   <223> Xaa can be any naturally occurring amino acid
<400> 12
   Gly Leu Glu Xaa Asn Pro Gly
   1               5
<210> 13
   <211> 8
   <212> PRT
   <213> Artificial
<220>
   <223> Peptide
<220>
   <221> misc_feature
   <222> (4)..(4)
   <223> Xaa can be any naturally occurring amino acid
<400> 13
   Asp Val Glu Xaa Asn Pro Gly Pro
   1               5
<210> 14
   <211> 8
   <212> PRT
   <213> Artificial
<220>
   <223> Peptide
<220>
   <221> misc_feature
   <222> (4)..(4)
   <223> Xaa can be any naturally occurring amino acid
<400> 14
   Asp Ile Glu Xaa Asn Pro Gly Pro
   1               5
<210> 15
   <211> 8
   <212> PRT
   <213> Artificial
<220>
   <223> Peptide
<220>
   <221> misc_feature
   <222> (4)..(4)
   <223> Xaa can be any naturally occurring amino acid
<400> 15
   Asp Leu Glu Xaa Asn Pro Gly Pro
   1               5
<210> 16
   <211> 8
   <212> PRT
   <213> Artificial
<220>
   <223> Peptide
<220>
   <221> misc_feature
   <222> (4)..(4)
   <223> Xaa can be any naturally occurring amino acid
<400> 16
   Gly Val Glu Xaa Asn Pro Gly Pro
   1               5
<210> 17
   <211> 8
   <212> PRT
   <213> Artificial
<220>
   <223> Peptide
<220>
   <221> misc_feature
   <222> (4)..(4)
   <223> Xaa can be any naturally occurring amino acid
<400> 17
   Gly Ile Glu Xaa Asn Pro Gly Pro
   1               5
<210> 18
   <211> 8
   <212> PRT
   <213> Artificial
<220>
   <223> Peptide
<220>
   <221> misc_feature
   <222> (4)..(4)
   <223> Xaa can be any naturally occurring amino acid
<400> 18
   Gly Leu Glu Xaa Asn Pro Gly Pro
   1               5
<210> 19
   <211> 16
   <212> PRT
   <213> Artificial
<220>
   <223> Peptide
<400> 19
   Asn Phe Asp Leu Leu Lys Leu Ala Gly Asp Val Glx Ser Asn Pro Gly
   1               5                   10                  15
<210> 20
   <211> 18
   <212> PRT
   <213> Artificial
<220>
   <223> Peptide
<400> 20
   Glu Gly Arg Gly Ser Leu Leu Thr Cys Gly Asp Val Glu Glu Asn Pro
   1               5                   10                  15
   Gly Pro
<210> 21
   <211> 25
   <212> PRT
   <213> Artificial
<220>
   <223> Peptide
<400> 21
   Gly Ile Phe Asn Ala His Tyr Ala Gly Tyr Phe Ala Asp Leu Leu Ile
   1               5                   10                  15
   His Asp Ile Glu Thr Asn Pro Gly Pro
               20                  25
<210> 22
   <211> 25
   <212> PRT
   <213> Artificial
<220>
   <223> Peptide
<400> 22
   Lys Ala Val Arg Gly Tyr His Ala Asp Tyr Tyr Lys Gln Arg Leu Ile
   1 5 10 15
   His Asp Val Glu Met Asn Pro Gly Pro
   20 25
<210> 23
   <211> 19
   <212> PRT
   <213> Artificial
<220>
   <223> Peptide
<400> 23
   Leu Leu Asn Phe Asp Leu Leu Lys Leu Ala Gly Asp Val Glu Ser Asn
   1 5 10 15
   Pro Gly Pro
<210> 24
   <211> 19
   <212> PRT
   <213> Artificial
<220>
   <223> Peptide
<400> 24
   Leu Thr Asn Phe Asp Leu Leu Lys Leu Ala Gly Asp Val Glu Ser Asn
   1 5 10 15
   Pro Gly Pro
<210> 25
   <211> 19
   <212> PRT
   <213> Artificial
<220>
   <223> Peptide
<400> 25
   Cys Thr Asn Tyr Ser Leu Leu Lys Leu Ala Gly Asp Val Glu Ser Asn
   1 5 10 15
   Pro Gly Pro
<210> 26
   <211> 20
   <212> PRT
   <213> Artificial
<220>
   <223> Peptide
<400> 26
   Gly Ala Thr Asn Phe Ser Leu Leu Lys Leu Ala Gly Asp Val Glu Leu
   1 5 10 15
   Asn Pro Gly Pro
   20
<210> 27
   <211> 22
   <212> PRT
   <213> Artificial
<220>
   <223> Peptide
<400> 27
   Gly Pro Gly Ala Thr Asn Phe Ser Leu Leu Lys Gln Ala Gly Asp Val
   1 5 10 15
   Glu Glu Asn Pro Gly Pro
   20
<210> 28
   <211> 20
   <212> PRT
   <213> Artificial
<220>
   <223> Peptide
<400> 28
   Phe Leu Arg Lys Arg Thr Gln Leu Leu Met Ser Gly Asp Val Glu Ser
   1 5 10 15
   Asn Pro Gly Pro
   20
<210> 29
   <211> 20
   <212> PRT
   <213> Artificial
<220>
   <223> Peptide
<400> 29
   Glu Ala Ala Arg Gln Met Leu Leu Leu Leu Ser Gly Asp Val Glu Thr
   1               5                   10                  15
   Asn Pro Gly Pro
               20
<210> 30
   <211> 20
   <212> PRT
   <213> Artificial
<220>
   <223> Peptide
<400> 30
   Gly Ser Trp Thr Asp Ile Leu Leu Leu Leu Ser Gly Asp Val Glu Thr
   1               5                   10                  15
   Asn Pro Gly Pro
               20
<210> 31
   <211> 20
   <212> PRT
   <213> Artificial
<220>
   <223> Peptide
<400> 31
   Thr Arg Ala Glu Ile Glu Asp Glu Leu Ile Arg Ala Gly Ile Glu Ser
   1               5                   10                  15
   Asn Pro Gly Pro
               20
<210> 32
   <211> 20
   <212> PRT
   <213> Artificial
<220>
   <223> Peptide
<400> 32
   Arg Ala Glu Gly Arg Gly Ser Leu Leu Thr Cys Gly Asp Val Glu Glu
   1 5 10 15
   Asn Pro Gly Pro
   20
<210> 33
   <211> 20
   <212> PRT
   <213> Artificial
<220>
   <223> Peptide
<400> 33
   Ala Lys Phe Gln Ile Asp Lys Ile Leu Ile Ser Gly Asp Val Glu Leu
   1 5 10 15
   Asn Pro Gly Pro
   20
<210> 34
   <211> 20
   <212> PRT
   <213> Artificial
<220>
   <223> Peptide
<400> 34
   Ser Lys Phe Gln Ile Asp Lys Ile Leu Ile Ser Gly Asp Ile Glu Leu
   1 5 10 15
   Asn Pro Gly Pro
   20
<210> 35
   <211> 20
   <212> PRT
   <213> Artificial
<220>
   <223> Peptide
<400> 35
   Ser Ser Ile Ile Arg Thr Lys Met Leu Val Ser Gly Asp Val Glu Glu
   1               5                   10                  15
   Asn Pro Gly Pro
               20
<210> 36
   <211> 20
   <212> PRT
   <213> Artificial
<220>
   <223> Peptide
<400> 36
   Cys Asp Ala Gln Arg Gln Lys Leu Leu Leu Ser Gly Asp Ile Glu Gln
   1               5                   10                  15
   Asn Pro Gly Pro
               20
<210> 37
   <211> 12
   <212> DNA
   <213> Artificial
<220>
   <223> LoxP Site
<400> 37
   ataacttcgt at 12
<210> 38
   <211> 13
   <212> DNA
   <213> Artificial
<220>
   <223> LoxP site
<400> 38
   ataacttcgt ata 13
<210> 39
   <211> 22
   <212> DNA
   <213> Artificial
<220>
   <223> LoxP site
<400> 39
   agcatacatt atacgaagtt at 22
<210> 40
   <211> 10
   <212> PRT
   <213> Artificial
<220>
   <223> Peptide tag
<400> 40
   Glu Gln Lys Leu Ile Ser Glu Glu Asp Leu
   1 5 10
<210> 41
   <211> 9
   <212> PRT
   <213> Artificial
<220>
   <223> Peptide tag
<400> 41
   Tyr Pro Tyr Asp Val Pro Asp Tyr Ala
   1 5
<210> 42
   <211> 6
   <212> PRT
   <213> Artificial
<220>
   <223> Peptide tag
<400> 42
   His His His His His His
   1 5
<210> 43
   <211> 54
   <212> DNA
   <213> Artificial
<220>
   <223> 2A peptide encoding sequence
<400> 43
   gagggcagag gaagtcttct aacatgcggt gacgtggagg agaatcccgg ccct 54
<210> 44
   <211> 54
   <212> PRT
   <213> Artificial
<220>
   <223> 2A peptide encoding sequence
<400> 44
   Gly Ala Ala Gly Gly Thr Ala Gly Gly Gly Gly Cys Ala Gly Thr Cys
   1               5                   10                  15
   Thr Ala Cys Thr Thr Ala Cys Cys Thr Gly Thr Gly Gly Ala Gly Ala
               20                  25                  30
   Thr Gly Thr Ala Gly Ala Ala Gly Ala Ala Ala Ala Cys Cys Cys Ala
           35                  40                  45
   Gly Gly Ala Cys Cys Gly
       50
<210> 45
   <211> 34
   <212> DNA
   <213> Artificial
<220>
   <223> LoxP site
<400> 45
   ataacttcgt atagcataca ttatacgaag ttat 34

## Description

### FIELD OF THE INVENTION

The present invention relates generally to the fields of medicine and life science research, in particular regenerative medicine, neurobiology, cell biology, and stem cell bioengineering. More particularly, it concerns methods and compositions for the expansion of somatic stem and progenitor cells, preferably neural stem cells and neural progenitor cells, in particular *in vivo.*

### BACKGROUND OF THE INVENTION

Stem cells of the adult body (somatic stem cells) can expand and generate cell biologically identical somatic stem cells while preserving the ability to differentiate, given the proper stimulus, to generate all cells of a given tissue. Stem cells can undergo two types of cell division. Proliferative division gives rise to at least one identical daughter cell endowed with stem cell properties. Differentiative division, on the other hand, produces only progenitor cells with limited self-renewal potential or terminally differentiated postmitotic cells.

Somatic stem cells are particularly important for renewal of tissues with high turnover such as skin, blood, or the intestinal epithelium, or may be involved in tissue-repair upon injury, which underlies great hopes for regenerative medicine (Orkin and Zon, 2008; Fuchs, 2009; Li and Clevers, 2010). Also organs with a particularly low turnover, such as the central nervous system (CNS), contain neural stem cells (NSC) that have the capacity to generate i) other NSC, ii) more committed neural progenitors (NPC) or, finally, iii) terminally differentiated cells such as neurons or glia (Kriegstein and Alvarez-Buylla, 2009). In fact, a great deal of interest recently arose form studies of stem cells in the nervous system, not only because of their importance for understanding neural development and brain function but also for their therapeutic potential in the treatment of neurodegenerative diseases (Lindvall and Kokaia, 2006; Okano et al., 2007).

Neural stem cells from the developing embryo or fetus can be expanded by different means. WO2005089420 A2 describes the addition of leukemia inhibitory factor (LIF) and several publications have reported similar effects after manipulation of transcription or trophic factors and several signaling moleculs such as basic fibroblast growth factor (bFGF) (Lukaszewicz et al., 2002), β-catenin (Chenn and Walsh 2002), Notch (Kageyama et al., 2008), cell cycle regulators such as cdk4/cyclinD1 (Lange et al., 2009) and many others factors (reviewed in Salomoni and Calegari 20 10).

In particular, even if different methods have been designed to purify and culture NSC *in vitro* (WO 93/01275, WO 94/09119, WO 94/10292, WO 94/16718) their manipulation generally leads to their rapid differentiation. For example, it has been demonstrated that bFGF and epidermal growth factor (EGF) are needed for expansion and maintenance of human fetal NSC and their rodent homologs (Vescovi and Snyder, 1999; Taupin et al., 2000; Okano and Temple, 2009). These NSC cultures are normally grown as free floating clusters of cells (neurospheres), but the neurospheres cannot proliferate indefinitely in the presence of bFGF and EGF alone and major concerns were recently raised on the various unphysiological conditions used in this, and other, culture systems (Reynolds and Vescovi, 2009; Woolard and Fine, 2009).

However, as perhaps the major obstacle in basic stem cell research and their use in therapy, adult stem cells, in contrast to stem cells of the developing embryo, revealed particularly difficult to expand both *in vitro* and *in vivo* (Lindvall and Kokaia, 2006; Okano et al., 2007; Orkin and Zon, 2008; Fuchs, 2009; Li and Clevers, 2010). This is probably due to the different cell biology of adult somatic stem cells in comparison to stem cells of the developing embryo, and in particular to their quiescent state. Similarly, progenitor cells are also difficult to expand because, even if they constantly progress through the cell cycle without entering quiescence, their proliferative potential is very limited leading to only few cell division before final consumption to generate differentiated, postmitotic cells (Lindvall and Kokaia, 2006; Okano et al., 2007; Orkin and Zon, 2008; Fuchs, 2009; Li and Clevers, 2010).

As an additional limitation to the study of somatic stem cells, they essentially stop proliferating *in vivo* by entering quiescence (Orkin and Zon, 2008; Fuchs, 2009; Li and Clevers, 2010; Salomoni and Calegari, 2010). In addition, attempts made *in vivo* to force somatic stem cells to leave quiescence and enter the cell cycle revealed unsuccessful leading to complete depletion of the stem cell pool both in the CNS and in hematopoietic stem cells (Cheng et al., 2000; Kippin et al., 2005; Orford and Scadden, 2008). In essence, to our knowledge no efficient system has yet been found to inducibly control the expansion of adult stem or progenitor cells as a means to ultimately increase the generation of their differentiated progeny.

Because of their low numbers, no direct examination of the biological and molecular properties or the physiological relevance of neural stem cells and neural progenitor cells in the adult was possible up to know.

### OBJECT OF THE INVENTION

Thus the object of the invention is to provide compositions and methods that allow the expansion of somatic stem and progenitor cells, in particular neural stem cells (NSC) and neural progenitor cells (NPC), *in vitro* and in particular *in vivo.*

### BRIEF SUMMARY OF THE INVENTION

The object of the invention is solved by a vector that contains genes encoding a cyclin dependet kinase (cdk) and a cyclin forming a G1-specific cdk/cyclin protein complex, preferably cdk4 and cyclinD1 (cycD1). Other preferred cdks are chosen from cdk6 and cdk2. Other preferred cyclins are chosen cyclinD2, cyclinD3 and cyclinE.

Preferred combinations of cdks and cyclins are chosen from:
- cdk4 and cyclinD1, cyclin D2 or cyclin D3,
- cdk2 and cyclinE,
- cdk6 and cyclinD1, cyclin D2 or cyclin D3.

Advantageously, the transfection or transduction of somatic stem cells by said vector allows the expansion of somatic stem cells (increasing the number of somatic stem cells) *in vitro* and in particular *in vivo* by increasing the proliferation of the transfected or transduced somatic stem cells without affecting their stem cells characteristics, or influencing their multipotency, thus without affecting their potential to differentiate. Applied to progenitor cells the vector will similarly lead to their expansion.

In an alternative way to carry out the invention the somatic stem cells (or progenitor cells) are expanded by exposing them to a composition containing a cdk and a cyclin forming a G1-specific cdk/cyclin protein complex preferably selected as described above. Here the proteins cdk and cyclin are preferably conjugated to a cell penetrating peptide (CPP). CPPs typically have an amino acid composition containing either a high relative abundance of positively charged amino acids (preferably 30 to 90% of the residues) such as lysine or arginine, or have sequences that contain an alternating pattern of polar/charged amino acids and non-polar, hydrophobic amino acids. These two types of structures are referred to as polycationic or amphipathic, respectively. Preferred CPPs have a length of 8 to 20 amino acid residues and are in particular chosen from tat-petides, model amphipathic peptides, transportans (derived from Galaninin) or poly-arginine, SynB (derived from Protegrin) und cis-γ-amino-L-Prolinhaltigen Peptiden. Examples are:
RQIKIWFQNRRMKWKK SEQ ID No. 1 (a penetratin),
KLALKLALKALKAALKLA SEQ ID No. 2 (amphipathic model peptide)
RKKRRQRRR SEQ ID No. 3 (atatpeptide).

Other CPPs are mentioned in Zhou et al., 2009 and Lange 2006 included by reference in this respect.

By exposing the somatic stem cells or progenitor cells to recombinant proteins conjugated to a short peptide, such as a HIV-tat or poly-arginine, cell transduction can be mediated and proteins delivered into cells (Zhou et al., 2009). Though the exact molecular mechanism underlying this transduction is not yet fully elucidated, proteins will by taken up by the somatic stem cells or progenitor cells resulting in a proliferation similar as described above.

This way for carrying out the invention is in particular suitable for *in vitro* expansion, but can also by applied *in vivo.*

Finally, equally effective will be the use and exposure to drugs or other compounds which main effect is to promote the expression and/or activity of cdk/cyclin complexes in G I thereby triggering similar effects as the ones described for the vector or protein composition. Preferred examples of compounds activating cdk/cyclins in G1 are estrogens (Prall OW et al. 1997, J Biol Chem. 272:10882-94).

During the effect induced by the invention, somatic stem cell and/or progenitor cells will divide to proliferate and generate more daughter cells that preserve their multipotency (thus the potential to forming more than one differentiated cell type). By using the invention somatic stem cells can unlimitedly expand and generate cell biologically identical somatic stem cells while preserving the ability to differentiate, given the proper stimulus, to generate all cells of a given tissue.

The term somatic or adult stem cell refers to any stem cell other than an embryonic stem cell or stem cell derived from embryo or fetus. Somatic stem cells have two properties: the ability to divide and create another cell like itself and also divide and create a cell more differentiated than itself. The term somatic stem exludes cells from embryo or fetus and includes cells that can be found in children, as well as in adults. However, in the invention somatic stem cells of adults are preferred. Somatic stem cells differ from pluripotent or totipotent embryonic stem cells in that they can not give rise to an organism and have lost the potential to differentiate into all three germ layers: endoderm, mesoderm and ectoderm. Somatic stem cells are able to generate a restricted number of lineages (multipotent) and may be referred to by their tissue of origin (e.g. bone marrow stem cells) or by their differentiation potential (hematopoietic stem cell, neural stem cell, mesenchymal stem cell, endothelial stem cell, etc.).

As the multipotency of the somatic stem cells is not altered by the invention, the somatic stem cells transfected or transduced with the vector used in the invention differ from the so called induced pluripotent stem cells (iPS cells).

The term progenitor cells refers to a type of partially differentiated, usually unipotent cell that has lost most or all of the stem cell multipotency. Usually progenitor cells are cells capable of differentiating into one or two closely related final differentiated cells types. Preferred examples of progenitor cells are hosen from pancreatic progenitor cells thymocytes, meiocytes, megakaryoblasts, promegakaryocytes, lymphoblasts, normoblasts, bone marrow precursor cells, progenitor cells from the periosteum that are capable to develop into osteoblasts and chondroblasts, myeloblasts, satellite cells found in muscles, endothelial precursor cells, melanoblasts, neural progenitor cells, oligodendrocyte progenitor cells and myeloid precursor cells. Most preferred are neural progenitor cells (NPC). As the invention relates to somatic cells, the term progenitor cells as used in the invention preferably does not include progenitor cells obtained from the embryo and preferably not from the fetus.

Preferred somatic stem cells used in the invention are chosen from neural stem cells, hematopoietic stem cells, mesenchymal stem cells, endothelial stem cells and any other tissue-specific stem cell of the skin, gut, muscles, hair follicle and so on.

Particularly preferred are neural stem cells (NSC), most preferred are neural stem cells from the adult central nervous system, in particular the brain (isolated or *in vivo*). NSC are defined as a cell with unlimited potential to generate identical NSC as well as differentiated cells of the neural lineage such as NPC, neurons or glia. NSC are defined also by cellular markers such as Sox2+, Nestin+, GFAP+ and Pax6+ and can be distinguished from NPC that express additional markers such as Tbr2+, DCX+, Tis21+ as well as from neurons (that are Sox2-, Nestin-, GFAP- and Pax6+) by yet other markers such as NeuN+, NeuroD+, calretinin+, calbinding+ and many others (reviewed in Kempermann, 2004; Zhao et al., 2008). As the invention relates to somatic cells, the term neural stem cells as used in the invention do not include so called neural stem cells obtained from the embryo and preferably not from the fetus.

In contrast, a NPC is defined as a cell with limited proliferation potential that can only generate differentiated cells such as neurons or glia. In this definition neuroblasts and terminally-committed progenitors are included.

The invention can be applied to both NSC and NPC. In both cases the expansion of the cells will result in an increase the number of neurons.

Further terms used in the description of the invention are defined as follows: a cycling cell is a cell progressing through the cell cycle until completion of mitosis. A quiescent cell is a cell whose cell cycle is temporarily arrested. A postmitotic cell is a cell whose cell cycle is irreversibly blocked and never divides. A newborn neuron is a postmitotic neuron up to two weeks old identified by doublecortin immunostaining. For further definitions see Smith, 2006; Salomoni and Calegari, 2010.

The vector used in the invention can in principle be a plasmid or an artificial chromosome, which are useful for *in vitro* transfection. *In vitro* means into a cell or tissue, that is in tissue culture and/or isolated from a multicellular organism.

Preferably the vectors are used to transfect or transduce somatic stem and/or progenitor cells, preferably NSC and/or NPC, in particular in the adult brain, *in vivo.* The term transfection is used for introduction of plasmid vectors into a target cell. For the introduction of viral vectors into the target cell the terms transduction and infection are used with the same meaning. *In vivo* means in a living adult animal, preferably a vertebrate, in particular a mammal, e.g. a rodent, a human or a non-human animal, preferably an animal that is used as an animal model for neuroscience research, e.g. mice, rats, rabbits, ferrets and non human primates. The preferred route for delivery of DNA vectors into tissues of the central nervous system *in vivo* is the generation and stereotaxic injection of viral particles but alternatives approaches are also possible such as *in vivo* electroporation (Barnabé-Heider et a., 2008). In particular for somatic stem and/or progenitor cells outside the central nervous system, many other routes of delivery are possible including aerosol sprays for the peripheral nervous system and mucosae of mouth, nose, trachea and respiratory tracts or direct injection, electroporation or application of viral or DNA vectors for the skin, blood and bone marrow.

The composition containing cdk and cyclin proteins or compounds are preferably applied in a similar manner.

For the invention it is preferred that the cdk and cyclin proteins form a 1:1 complex, thus a protein complex, in which both proteins are present in a similar or preferably equimolar ratio. Other solutions may include the overexpression or activation of only one of the two proteins, the cdk or the cyclin, but the presence of both at 1:1 ratio to form a cdk/cyclin complex will increase efficiency and magnitude of the desired effect. This is preferably achieved by an equimolar or approximately equimolar overexpression of the cdk and the cyclin by the vector or by providing the proteins in the composition in an approximately equimolar ratio. Approximately meaning that one protein may be present in excess of 50 mol-%, preferably 20 mol-%, most preferred 10 mol-% in relation to the other.

In particular for *in vivo* applications preferably a lentiviral vector or herpes virus based vector is used in the invention for the transduction of stem cells. The lentiviral vector is preferably a self inactivating vector that can infect dividing and non-dividing cells, but is not capable of producing virus particles in the infected cells as the vector lacks viral genes necessary for virus production. The virus particles used for infection are produced in packaging cells expressing those genes necessary for virus production. For progenitor cells, in particular NPC, a retroviral vector, in particular a Moloney murine leukemia-derived genetically modified, replication incompetent gammaretroviruses, is preferred. However, a lentiviral vector or herpes virus based vector can also be applied.

For *in vitro* applications viral vectors such as lentiviral or herpes virus based vector are preferred. Here, however, the term vector includes also any other transfection system with plasmids or DNA/RNA fragments, delivery of proteins or, finally, use of drugs acting specifically on the expression or activity of cdk/cyclin complexes.

Preferably the vector comprises a fusion gene wherein the genes encoding the cdk and the cyclin are separated by a 2A peptide encoding sequence (DeFelipe et al., 2005). Specifically more than thirty different 2A self-cleaving peptide sequences, which are 15 to 25 amino acids residues long, are know (see http://www.st-andrews.ac.uk/ryanlab/Index.htm for a detailed list) but due to degeneration of the DNA-to-protein translation code, literally millions of DNA fragments for 2A peptides can be generated (DeFelipe et al., 2005). So, in essence, any of these sequences for DNA vectors would allow correct processing and generation of multiple proteins, in particular cdk/cyclins as for this invention, from a single mRNA.

Preferred 2A peptides are chosen from peptides containing one of the following peptide motives:

| | |
|---|---|
| ExNPG | SEQ ID No. 4 |
| DxExNPG | SEQ ID No. 5 |
| GxExNPG | SEQ ID No. 6 |
| DVExNPG | SEQ ID No. 7 |
| DIExNPG | SEQ ID No. 8 |
| DLExNPG | SEQ ID No. 9 |
| GVExNPG | SEQ ID No. 10 |
| GIExNPG | SEQ ID No. 11 |
| GLExNPG | SEQ ID No. 12 |
| DVExNPGP | SEQ ID No. 13 |
| DIExNPGP | SEQ ID No. 14 |
| DLExNPGP | SEQ ID No. 15 |
| GVExNPGP | SEQ ID No. 16 |
| GIExNPGP | SEQ ID No. 17 |
| GLExNPGP | SEQ ID No. 18 |

and preferably having a length of 15 to 25 amino acid residues, most preferred 18 to 20 amino acid residues, with x being 1 to 3 amino acid residues, preferably I amino acid residue, preferably encoding naturally occurring amino acids. Most preferred 2A peptides are chosen from:

| | |
|---|---|
| NFDLLKLAG**DV**ZS**NPG** | SEQ ID No. 19 |
| EGRGSLLTCG**DVE**ENPGP | SEQ ID No. 20 |
| GIFNAHYAGYFADLLIHDIETNPGP | SEQ ID No. 21 |
| KAVRGYHADYYKQRLIHDVEMNPGP | SEQ ID No. 22 |
| LLNFDLLKLAGDVESNPGP | SEQ ID No. 23 |
| LTNFDLLKLAGDVESNPGP | SEQ ID No. 24 |
| CTNYSLLKLAGDVESNPGP | SEQ ID No. 25 |
| GATNFSLLKLAGDVELNPGP | SEQ ID No. 26 |
| GPGATNFSLLKQAGDVEENPGP | SEQ ID No. 27 |
| FLRKRTQLLMSGDVESNPGP | SEQ ID No. 28 |
| EAARQMLLLLSGDVETNPGP | SEQ ID No. 29 |
| GSWTDILLLLSGDVETNPGP | SEQ ID No. 30 |
| TRAEIEDELIRAGIESNPGP | SEQ ID No. 31 |
| RAEGRGSLLTCGDVEENPGP | SEQ ID No. 32 |
| AKFQIDKILISGDVELNPGP | SEQ ID No. 33 |
| SKFQIDKILISGDIELNPGP | SEQ ID No. 34 |
| SSIIRTKMLVSGDVEENPGP | SEQ ID No. 35 |
| CDAQRQKLLLSGDIEQNPGP | SEQ ID No. 36 |

The transcription of the fusion gene results in a bi- or polycistronic expression, meaning that the fusion gene is transcribed into a single messenger RNA. The 2A peptide sequence results in the co-translational cleavage of proteins. They are more reliable than classical internal ribosomal entry sites and lead to expression of the single genes multiple cistrons at equimolar levels. Thus, the fusion gene with the 2A peptide encoding sequence advantageously results not only in a simultaneously but equimolar overexpression of the cdk, the cyclin proteins and, eventually, any other third, or fourth gene such as an expression reporter, a selection marker or recombination gene.

The fusion gene is under the control of either a constitutive or inducible promoter. Preferably a constitutive promoter is chosen, most preferred the ubiquitin promoter. In particular when a constitutive promoter is used the fusion gene (encoding the cdk and the cyclin) is preferably inserted as an inducible knock on or inducible knock out construct. The inducible knock out has the advantage that the fusion gene can be knocked out under specific well defined conditions, eliminating or deactivating the fusion gene (encoding the cdk and the cyclin). Consequently the stem cells stop proliferating and start to differentiate after knock out. Similar the inducible knock on has the advantage that the fusion gene can be activated (knocked on) under specific well defined conditions.

Inducible knock on and knock out constructs are know from the literature. A preferred example is the Cre-lox system. In the Cre-lox system a stop cassette (knock on) or the fusion gene (encoding the cdk and the cyclin) (knock out) is flanked by lox-sites (one up-stream, one down-stream) thereby allowing to activate, or inactivate, cdk/cyclin expression by removal of the stop, or cdk/cyclin, cassette respectively.

The lentiviral vector results in an insertion of the fusion gene into the genome of the infected cell. The lox-sites advantageously allow the inactivation or excision of the fusion gene by an enzyme called Cre recombinase. The lox-sites are preferably both in the same orientation, so that Cre recombinase performs an excision and eliminates the fusion gene from the infected cells. If the lox-sites are in opposite orientation to each other Cre recombinase inactivates the fusion gene by performing an inversion. Thus, by bringing the fusion gene into contact with the Cre protein, the fusion gene is either eliminated or inactivated. Consequently the cells stop proliferating.

Preferred lox sites are chosen from loxP (ATAACTTCGTAT - SEQ ID No. 37, ATAACTTCGTATA - SEQ ID No. 38, reverse sequences and multimers thereof, like AGCATACATTATACGAAGTTAT - SEQ I D No. 3 9), x2272, and loxN. Several systems have been developed that allow the inducible activity of Cre protein.

Other systems are based on different DNA recombinases (further referred to as recombinases), preferably chosen form other Tyrosine site-specific recombinases (SSRs), such as Dre (Anastassiadis et al., 2009) or Tre (Buchholz, 2009). A preferred alternative to the Cre-lox-System is the Dre-rox system (Anastassiadis et al., 2009).

Another preferred example for such an inducible knock out system is the Tet-Off system, which is comprised of a tet transactivator tTA and a tetO responsive Tyrosine site-specific recombinases (SSRs), such as Cre recombinase. Binding of tTA, in the absence of doxycycline (dox), to the tet promoter causes expression of the SSR, e. g. Cre. Thus the fusion gene (encoding cdk4 and cycD1) will only be active in the presence of doxycycline (dox) or another tetracycline analogue.

Another preferred example for an inducible knock on system is the Tet-On system, which is comprised of a tet transactivator tTA and a tetO operator. In this system the rtTA protein is only capable of binding the operator when bound by doxycycline or another tetracycline analogue. Once bound the 'tet'O operator will activate a promoter coupled to the 'tet'O operator, activating the transcription of the fusion gene (encoding the cdk and the cyclin). Thus the fusion gene (encoding cdk4 and cycD1) will only be active in the presence of doxycycline (dox) or another tetracycline analogue. The Tet-On system has a faster responsiveness than the Tet-Off system.

In other inducible knock out systems the recombinase, e.g. an SSR like Cre, encoding gene is placed under control of an inducible promoter. In a preferred system the gene encoding the recombinase is fused to a gene encoding a hormone binding domain of a receptor (further referred to as receptor fusions), preferably a hormone binding domain of a steroid receptor or a mutated version thereof. Receptor fusions are preferably selected from CreERt or CreER^{T2} (Cre fused to the ligand binding domain of a mutated human estrogen receptor that recognizes anti-estrogen 4- hydroxyltamoxifen), Mer-CreMer or CreP (Cre fused to the progesterone receptor) or DreP (Dre fused to the progesterone receptor). In these systems SSR (in particular Cre or Dre) activity can be induced by administering substances binding to the hormone binding domain of the receptor fusion e.g. administering 4- hydroxyltamoxifen or progesterone.

These systems can be applied in the invention in several ways:
1. Co-transfection or co-transduction the somatic stem and/or progenitor cells, in particular NSC and/or NPC, with a construct allowing the induction of activity of a recombinase, preferably an SSR like Cre, Dre or Tre;
2. Transfecting or transduction of somatic stem and/or progenitor cells, in particular NSC and/or NPC, that already contain a construct allowing the induction of activity of a recombinase, preferably an SSR like Cre, Dre orTreCre protein;
3. A second transfection or transduction of the somatic stem and/or progenitor cells, in particular NSC and/or NPC, with a vector encoding a recombinase, preferably an SSR like Cre, Dre or Tre.

In the first case the recombinase encoding gene is preferably inserted into the same vector as the fusion gene - preferably as a separate gene. When a receptor fusion is chosen as recombinase, this receptor fusion can be either on a separate gene or as part of the fusion gene (encoding the cdk and the cyclin) preferably separated by an 2A peptide.

In the second case preferably somatic stem cells, in particular NSC and/or NPC, are used that are previously transduced with a construct allowing the inducible expression of the recombinase. Alternatively the invention is performed *in vivo* on animals that are transgenic for a construct allowing the inducible expression of the recombinase in all cells or at least in the somatic stem cells and/or progenitor cells, in particular NSC and/or NPC. Another option is to use somatic stem cells and/or progenitor cells, in particular NSC and/or NPC isolated from these transgenic mice *in vitro.*

When a receptor fusion, like CreERt or CreER^{T2}, is used the recombinase activity can advantageously be induced by the administration of a drug, like 4-hydroxyltamoxifen (4-OHT). Thus, by bringing the fusion gene into contact with the recombinase, the fusion gene is either eliminated or inactivated. Consequently the cells stop proliferating.

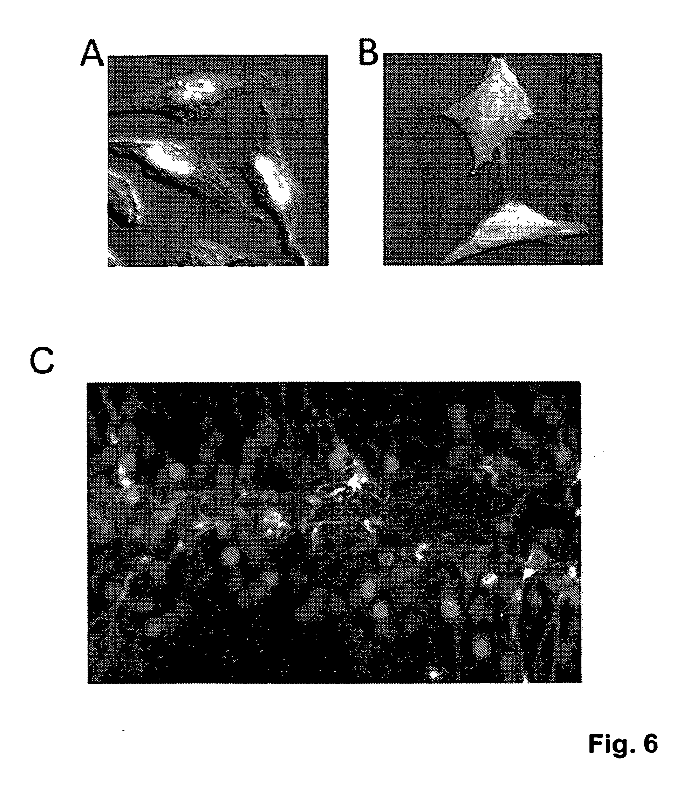

It has been shown by the inventors that an orally administered or intraperitoneally injected drug, like 4-OHT, can induce recombinase activity in somatic stem cells and/or progenitor cells, in particular NSC and/or NPC *in vivo* in the adult brain, leading to an inactivation of the fusion gene (encoding the cdk and the cyclin) (Fig 6) and the cells to stop proliferating.

Especially for *in vitro* applications transient transfections or transfections in which transfected genes remain episomal and/or their protein products can be removed overtime by direct manipulation or simply by dilution over consecutive divisions are an alternative to the inducible constructs (e.g. knock on or knock out constructs).

Especially for use in research the vector additionally contains a gene encoding a reporter protein and/or a sequence encoding an epitope tag or any other reporter for distinguishing transfected cells from non transfected including for example, selection and survival genes providing resistance to toxic molecules.

A reporter protein is one that can easily be detected preferably by measuring fluorescence, light emission or a color reaction or any other reporter for distinguishing transfected cells from non transfected. Preferred reporter proteins chosen from fluorescent proteins (like GFP), luciferase or β-galactosidase (encoded by the bacterial gene lacZ). An epitope is a portion of a molecule to which an antibody binds. Preferred epitope tags are peptides with a length of range from 10 to 15 amino acids long and are designed to easily detect a protein. Non limiting examples include c-myc (EQKLISEEDL - SEQ ID No. 40), HA (YPYDVPDYA -SEQ ID No. 41) and His6 (SEQ I D No. 42 - HHHHHH). The gene encoding the reporter protein and/or the sequence encoding an epitope tag is preferably also part of the fusion gene (encoding the cdk and the cyclin) and preferably separated from the genes encoding the cdk and the cyclin by an 2A peptide encoding sequence. Preferably the cleaved fragment of 2A peptides fused to cdk or cyclin can themselves is used as a tag.

Another object of the invention are somatic stem cell and/or progenitor cells, in particular NSC and/or NPC transfected or transduced by a vector as defined in the invention.

In another aspect the invention comprises an animal model wherein somatic stem cells and/or progenitor cells, in particular NSC, or NPC are transfected or transduced by a vector as defined in the invention. A preferred example is transgenic mice for the inducible expression of cdk and a cyclin allowing the inducible expansion of stem cells and progenitor cells. This is achieved by combining available technologies for inducible control of gene expression *in vivo*, preferably as described above. Advantageously, transgenic animals allow to directly influence the fate of all cells of a given tissue and not only the subpopulation that is being targeted by viral infection or the other methods described above.

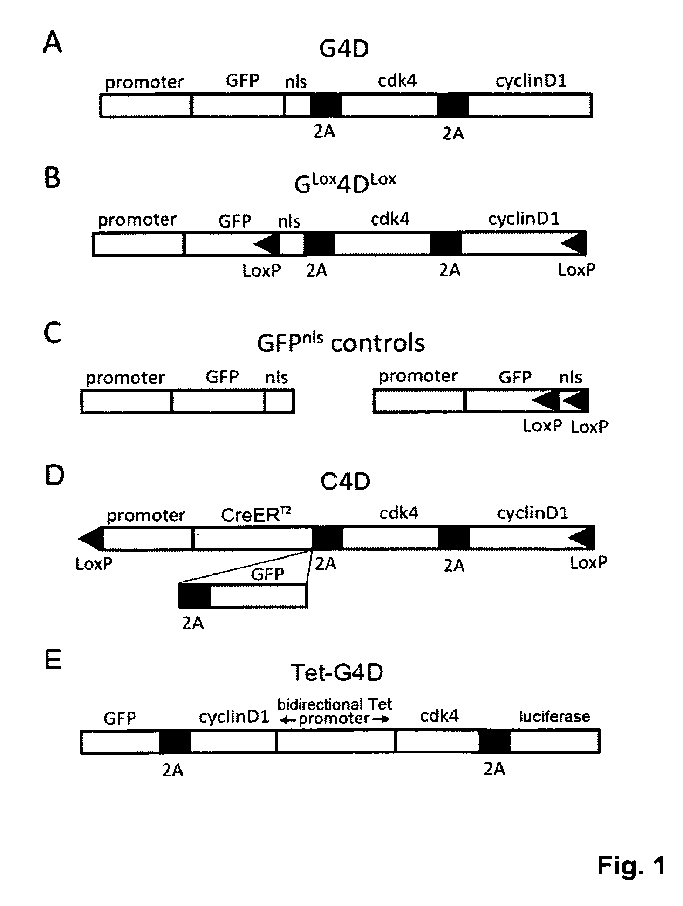

In a preferred example, using the Doxycycline-inducible system the inventors have generated tissue-specific, tetracycline-inducible (Tet-On) cdk/cyclin mouse lines for the temporal and spatial manipulation of cdk/cyclin expression in NSC thus controlling their proliferation vs. differentiation during embryonic development or adulthood. Specifically transgenic mouse lines are preferred in which a bidirectional Tet-promoter drives simultaneous expression of, in one direction, a cyclin and a GFP and, in the other direction, a cdk and a gene encoding a reporter, as luciferase, which expression can advantageously be used for precise quantification of responsiveness upon Doxycycline administration (Fig 1E). Clearly, the GFP and/or the luciferase can be replaced by any other desired gene for cell selection, identification or genetic recombination as described above. Importantly, these Tet-On responder mouse lines were crossed with several other transgenic mouse lines in which Tet expression is under the control of cell-specific promoters. These bi-genic mice (and any other additional crossing of the responder with any Tet expressing line), allows to overcome the only limitation of the vector transduction systems described until now, which, as efficient as they can be, only allow transduction in a in subpopulation (relatively small) of cells rather than in all the relevant cells of a given tissue.

Advantageously the responder Dox-inducible cdk/cyclin mouse lines generated by the inventors can be crossed with any of the many available tissue-specific Tet-On activator lines thus allowing the expansion/differentiation of any somatic stem or progenitor cell *in vivo* by simple addition/removal of Doxycycline.

If applied to NSC or NPC, this system advantageously allows to trigger much greater effects on expansion that can be applied for a multitude of different purposes and contexts e.g.: during i) embryonic development, ii) adulthood, or iii) in mouse model of neurodegenerative diseases or CNS injuries.

Expansion of NSC or NPC during adulthood allows addressing the hitherto elusive role of adult neurogenesis for elaborate function such as memory and learning. Expansion of NSC or NPC in models of CNS disease or injury can be use to establish platforms for the investigation of novel approaches of regenerative therapies in animal models.

The invention also comprises a method for the expansion of somatic stem cells and/or progenitor cells, in particular NSC and/or NPC with the steps:
a.) providing a vector as defined in the invention,
b.) introducing said vector into somatic stem cells and/or progenitor cells by transfection or transduction,
c.) overexpression of cdk and the cyclin protein complex in the transfected or transduced somatic stem cells and/or progenitor cells, resulting in proliferation of the transfected or transduced somatic stem cells and/or progenitor cells.

In an alternative way to carry out the invention comprises a method for the expansion of somatic stem cells and/or progenitor cells, in particular NSC and/or NPC, with the steps:
a') contacting somatic stem cells with a protein composition containing cdk and a cyclin as defined above or a compound as defined in the invention resulting in proliferation of the somatic stem cells and/or progenitor cells.

Step b.) is either carried out *in vitro* or in particular *in vivo* by applying the vector to an animal as described above. Alternatively step a') is carried out by applying the composition or compound to isolated cells *in vitro* or *in vivo* to an animal as described above.

The invention comprises also somatic stem cells and/or progenitor cells, in particular NSC and/or NPC, prepared by the method described above. The somatic stem cells and/or progenitor cells transfected or transduced with the vector according to the invention contain either the vector or after induced knock-out remaining (flanking) sequences of the vector.

A preferred way of carrying out the method of the invention comprises the additional step of an induced knock out of the overexpressed cdk and the cyclin genes, preferably by inducing the recombinase activity, after the number of adult neural stem have been increased (step c.) of the method of the invention). As described above the induced knock out, preferably by inducing recombinase activity, will excise or inactivate the cdk and the cyclin genes in the transfected or transduced cells. Consequently the cdk and the cyclin induced proliferation of the cells (stem cells and/or progenitor cells) will be stopped.

Alternatively the protein composition containing cdk and a cyclin or compound is supplied as long as the stem cells and/or progenitor cells shall expand. Here the proliferation is simply stopped by not further supplying the composition or by rinsing the cells.

Similar in case of a knock on construct (like the Tet-On system) the drug inducing the gene expression (e. g. tetracycline) is applied as long as the stem cells and/or progenitor cells shall expand and the expression is stopped by not further supplying the drug (e.g. tetracycline).

In Summary the inventions provides a system that allows:
1. a controlled increase of the somatic stem cell population and/or progenitor cell population, preferably NSC and/or NPC, in particular *in vivo* (in the adult brain) by inducing their proliferation,
2. to stop the proliferation of the somatic stem cell population and/or progenitor cell population (in particular NSC and/or NPC), which allows the somatic stem cells and/or progenitor cell population,to differentiate and to regenerate tissue, in case of NSC and/or NPC to perform neurogenesis or gliogenesis.

Consequently regeneration of tissue, e.g. neurogenesis, is increased by the invention by increasing the somatic stem cell population and/or progenitor cells. Advantageously, the overexpression of the cdk and the cyclin protein complex is not toxic and increases proliferation, without affecting the multipotency of the somatic stem cells, in particular NSC. A further advantage of the system of the invention is that the induced proliferation does not lead to tumor development or carcinogenesis. As the overexpression of the cdk and the cyclin can be stopped efficiently (e. g. by inducing the knock out of the overexpressed cdk and the cyclin genes) the system is safe.

The invention is useful for life science, neuroscience and/or pharmaceutical research, in particular to study the role and function of somatic stem cells and/or progenitor cells, in particular NSC and/or NPC in the adult brain. Further the invention is preferably applied to animal models for diseases, disorders or conditions of the central nervous system.

Also expansion of somatic stem cells and/or progenitor cells, in particular NSC and/or NPC, *in vitro* can advantageously be used for various purposes in basic research, diagnosis or in cell-based therapeutic applications by cell transplantions or any other means.

The observation that exit from quiescence and entry in the cell cycle can be induced by cdk/cyclin overexpression is unprecedented. Importantly, since somatic stem cells during adulthood are known to be quiescent in essentially all tissues, it follows that the invention can be applied to any other somatic stem cell such as hematopoietic or stem cells of the skin, which can be targeted by various means as already described.

For instance, bone marrow transplantation is a common and well established approach in many therapeutic applications of hematopoietic stem cells (HSC). However, the major limiting factor of this technique is the very limited amount of bone marrow that can be collected from a compatible donor. Thus, expansion of HSC, prior to of following transplantation, will allow to greatly improve the efficiency of stem cells based therapies. In essence, transduction of HSC with cdk/cyclins vectors can be preformed either *in vivo* by direct injection in the bone marrow or, alternatively, *in vitro* upon removal of bone marrow tissue from a donor to be later transfused in a receiving patient or back to the donor. In particular for the later approach, expansion of HSC by transduction of cdk/cyclin vectors *in vitro* seems preferable as this will allow subsequent tamoxifen treatment and Cre-mediated recombination to occur outside the patient clearly avoiding any potential side effect due to tamoxifen administration *in vivo.* In this case, a fluorescent reporter, such as GFP, is preferably also inserted together with the recombinase (like CreER^{T2}) using 2A sequences as this will allow to remove by FAC-sorting the few cells in which recombination may eventually not occur due to intrinsic limitations of the various recombination systems.

The use of vectors as defined in the invention (like CreER^{T2}/GFP/cdk/cyclin) is thus the preferred approach for the inducible and temporally controlled expansion of any somatic stem cell which allows manipulations to be performed completely *in vitro* while simultaneously allowing selection of absolutely pure population of cells for transplantation that are completely devoid of any exogenous DNA.

The invention also comprises the vector or composition or compound as defined in the invention for several possible applications in basic scientific research and their use in the expansion of somatic stem cells and/or progenitor cells, in particular NSC and/or NPC, for therapies for the treatment of a human or an animal having a disease, disorder or condition of the central nervous system (CNS), preferably selected from the group consisting of a neurodegenerative diseases, developmental disorders of the CNS, or injury of the CNS caused by accidents or tumors, in particular Huntington's disease, Alzheimer's disease, Parkinson's disease, brain injury or trauma, spinal cord injury or trauma, stroke, multiple sclerosis, cancer, CNS lysosomal storage diseases and head trauma and epilepsy.

The invention includes a method of treating a human or an animal having a disease, disorder or condition of the CNS. The disease, disorder or condition of the CNS is preferably selected from the group consisting of a neurodegenerative diseases, developmental disorders of the CNS, or injury of the CNS caused by accidents or tumors, in particular Huntington's disease, Alzheimer's disease, Parkinson's disease, brain injury or trauma, spinal cord injury or trauma, stroke, multiple sclerosis, cancer, CNS lysosomal storage diseases and head trauma, epilepsy and any other malfunctions of the CNS.

In yet another aspect, the disease, disorder or condition is injury to the tissue or cells of the CNS, which might be the result of the removal of a brain tumor or cancer.

The treatment is preferably carried out by applying a vector or composition defined according to the invention to the human or animal as defined above to expand somatic stem cells and/or progenitor cells, in particular NSC and/or NPC, *in vivo.* In another aspect, somatic stem cells and/or progenitor cells, in particular NSC and/or NPC are isolated from the human or animal or differentiated from other cells and expanded *in vitro* by transforming or transducing them with a vector as defined in the invention or by contact with the composition as defined in the invention. *In vitro* expanded cells are administered to the CNS. Alternatively, cells are transformed or transduced *in vitro* and administered directly to the CNS and replicate in the CNS.

Viral infection is one of the most intensely studied approach for gene therapy in humans thank to the characterization of several strains of non pathogenic viruses (Cameron and McKay, 2001; Lowenstein and Castro, 2002) and, thus, the findings of the invention are directly relevant for therapy in the human CNS. For the infection of NSC and/or NPC in the human brain the vector contains preferably additionally to the fusiongene encoding the cdk/cyclin complex a gene encoding a recombinase, preferably a receptor fusion as described above to mediate the removal of the exogenous DNA from human cell at the desired time by administration of a drug. In essence the vector is designed as to allow its efficient removal at any time point after infection.

It is important to stress that while many tumors were found to overexpress cyclins and cdks, the overexpression of the cdk/cyclin complex used in the invention was not shown to cause tumorogenesis. This was confirmed both in the embryo as well and in the adult, were no obvious formation of tumors were observed at any stage of analyses. Groups of mice were subjected to cyclin and cdk overexpression for several months to investigate this important issue. Further, the possibility to conditionally remove (or knock out) the cyclin and cdk genes allows to minimize, if not completely exclude, possible long term side effects of the ectopic genes.

### EXAMPLES

The following figures and examples are presented in order to more fully illustrate the preferred embodiments of the invention. These examples should in no way be construed as limiting the scope of the invention.
**Fig. 1** shows the maps of the viral constructs for (**A**) G4D, (**B**) G^{Lox}4D^{Lox}, and respective (**C**) GFP^{nls} controls (left and right, respectively). Similar 2A peptides (T2A with degenerated DNA sequence) and promoters (CMV) were used. The black triangles represent the loxP sites. (**D**) map of a construct to be used in therapy (C4D) in which GFP has been replaced by, or is inserted in addition to (inset indicated by lines), an inducible CreER^{T2} and LoxP sites were inserted as to remove the whole exogenous vector upon tamoxifen administrartion. (**E**) map of a vector used for the generation of Tet-responder transgenic mice for the inducible, Doxycycline-dependent expression of cdk/cyclins/GFP/luciferase. These mice were crossed with tissue-specific Tet-expressing mice (not shown).

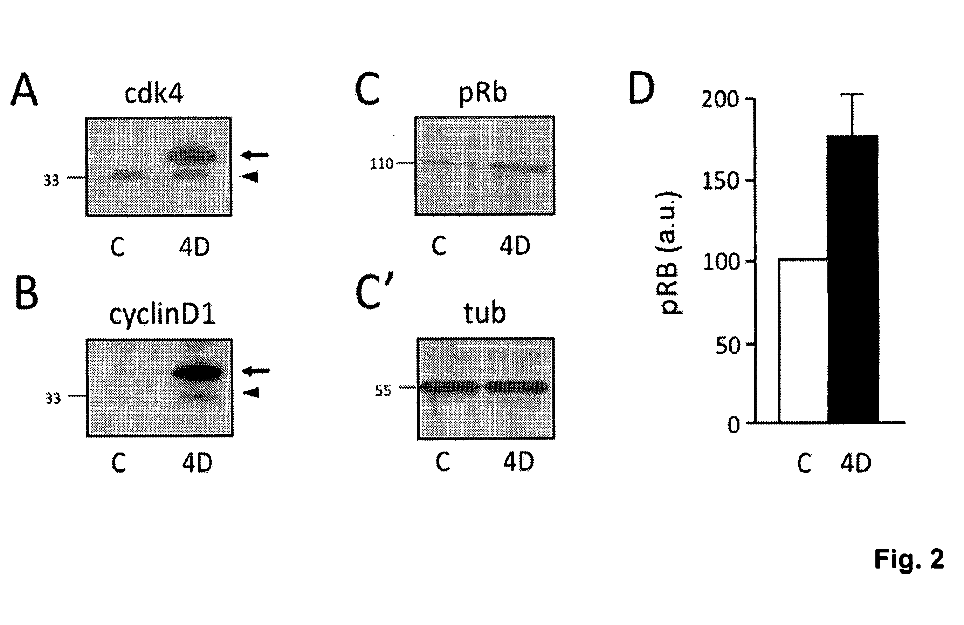

**Fig. 2** shows the viral-mediated expression of catalytically active 4D (**A-C**') in Western blot analyses of HeLa cells infected with 4D-overexpressing viruses after immunostaining with cdk4 (**A**), cyclinD1 (**B**), phosphorylated-specific Rb (**C**), or tubulin (**C'**) antibodies compared to GFP^{nls} control (C). Note the presence of two bands for cdk4 (**A**) and cyclinD1 (**B**) representing the endogenous cdk4 or cyclinD1 (arrowhead) or fusion products with 2A peptides (arrow). (**D**) Quantification of phosphorylated Rb after tubulin normalization as in **C** and **C'**.

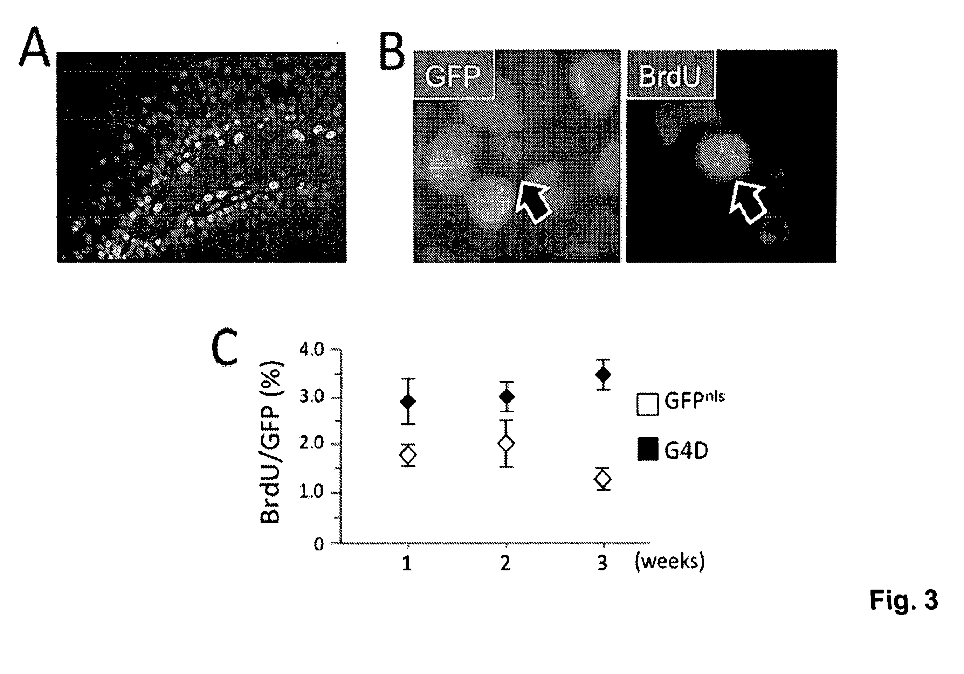

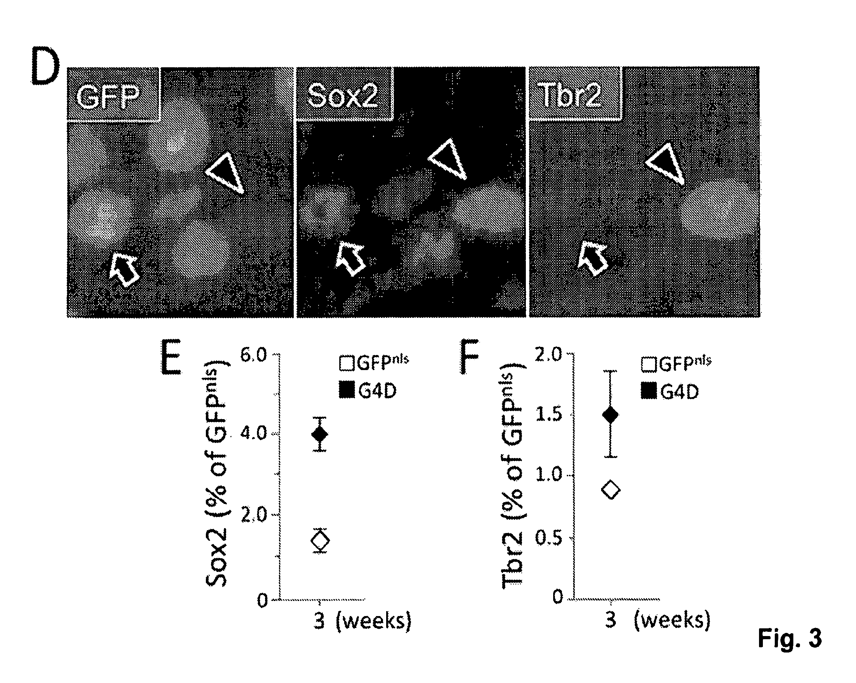

**Fig. 3** demonstrates that expression of G4D by lentiviral infection induces the expansion of NSC/NPC and inhibits neurogenesis: (**A** and **A'**) Fluorescent pictures of the mouse hippocampus infected with G4D lentiviruses showing individual infected cells (B) and DAPI staining (**A'**). (**B**) High-power view as in A after staining for GFP (left), BrdU (right. Arrows indicate a GFPBrdU+ cell. (**C**) Quantifications as in C at 1, 2, or 3 weeks after infection with GFP^{nls} (white boxes) and G4D (black boxes). (**D**) High-power view as in A after staining for GFP (left), Sox2 (middle), and Tbr2 (right) . (**E** and **F**) Quantification of Sox2 (**E**) and Tbr2 (**F**) as in D at 3 weeks from infection with GFP^{nls} (white boxes) and G4D (black boxes).

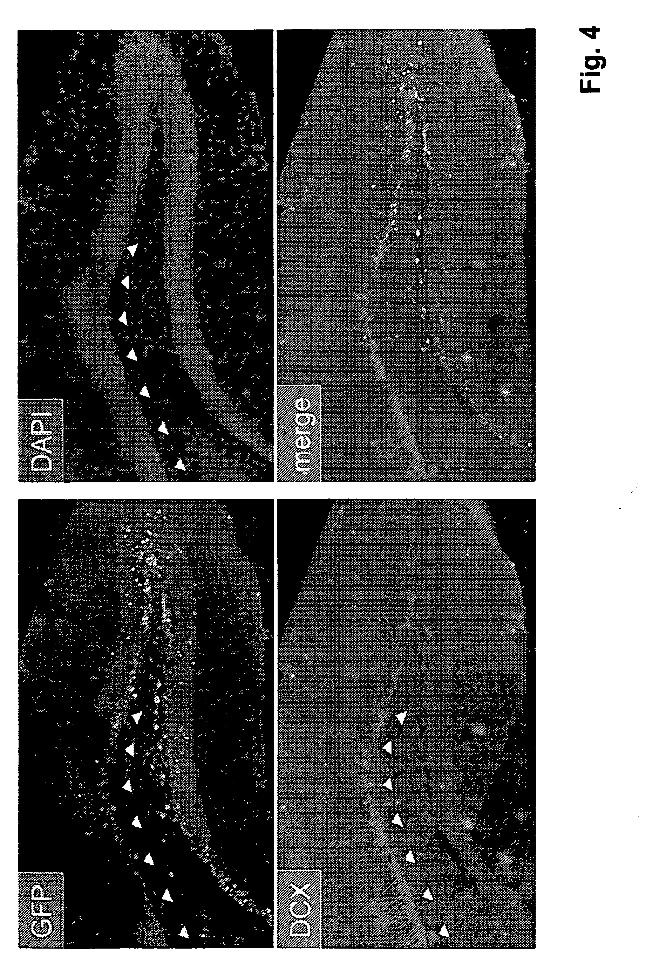

**Fig. 4** shows fluorescence pictures of the mouse hippocampus infected with G4D lentiviruses by detecting GFP (upper left), DCX (lower left), DAPI (upper right) immunoreactivity, or merged (lower right). The increased density of DCX+ cells in the uninfected portion of hippocampus is indicated by arrowheads.

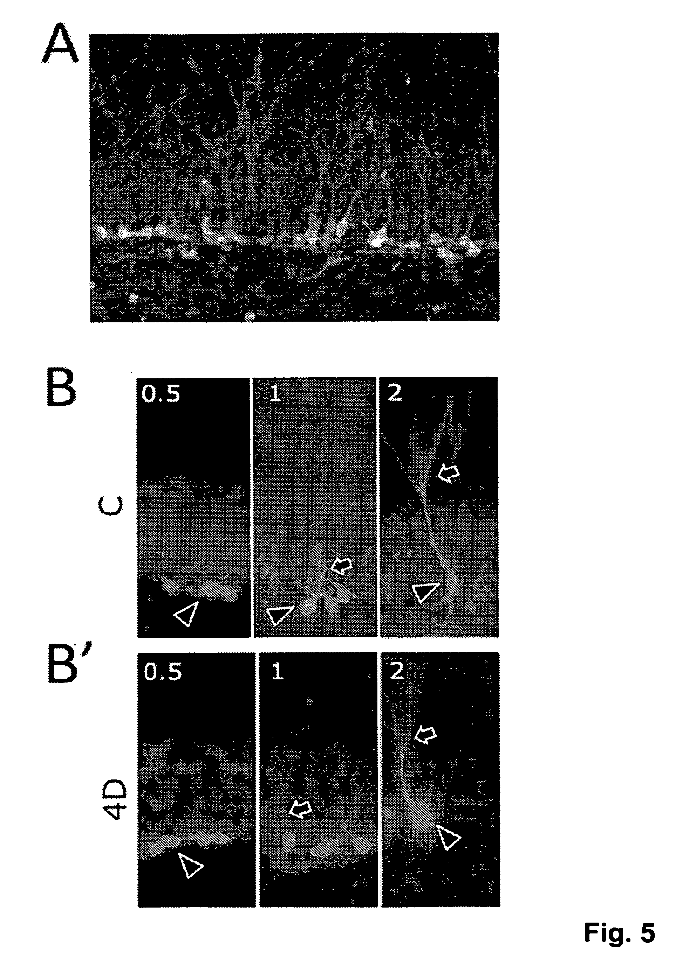

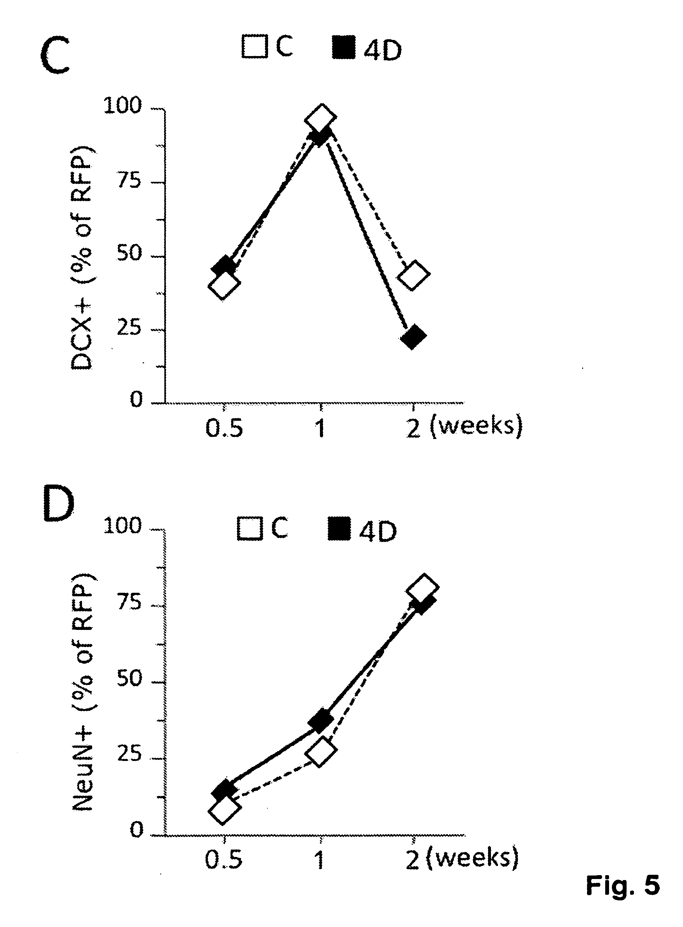

**Fig. 5** demonstrates that expression of G4D by MLV retrovirus infection has no effect on newborn neurons. (A) Fluorescence picture of the mouse hippocampus after infection with 4D-overexpressing and RFP expressing MLV. (B and B') High power view as in A showing the morphology of newborn neurons 0.5, 1, or 2 weeks after infection with control MLV (B; up) or 4D-overexpressing MLV (B'; bottom) particles. (C and D) quantification of DCX+ (C) or NeuN, a marker of more mature neurons, (D) after infection with RFP, control MLC (C, white symbols) or 4D-overexpressing MLV (4D, black symbols) particles. None of the differences are significant. The MLV enters in a mitotic NPC. This infected NPC soon after divides to generate neural cell daughters. Thus, expression of cdk/cyclin starts in the newborn neuron and NOT in the mother NPC. These data are important to exclude the possibility that overexpression of cdk/cyclin is toxic to neurons
**Fig. 6** shows the results of conditional excision of the G^{Lox}4D^{Lox} cassette (**A** and**B**). Nuclear (**A**) and cytoplasmic (**B**) localization of GFP after infection with G^{Lox}4D^{Lox}-expressing viruses in HeLa cells wild type (**A**) or HeLa cells expressing Cre recombinase (**B**). (**C**) fluorescent pictures showing redistribution of GFP fluorescence to the cytoplasm upon injection of G^{Lox}4D^{Lox}-expressing MLV in *nestin*^{CreERT2/-} mice followed by tamoxifen administration for 4 days.

### Example 1: transduction of NSC and NPC in vivo in mice

### Constructs.

To manipulate gene expression in NSC and NPC *in vivo* the inventors generated and stereotaxically injected i) HIV-derived genetically modified, replication incompetent lentiviruses (from now on referred to as HIV) or ii) Moloney murine leukemia-derived genetically modified, replication incompetent gammaretroviruses (from now on referred to as MLV). Upon infection, the viral genome irreversibly integrate into the genome of the host cells with the critical difference that MLV cannot transduce non mitotic cells because the viral integration complex can entry into the nucleus only if the nuclear envelope of the infected cell is disassembled, which only occurs in mitosis (Roe et al., 1993). Thus, while HIV allows the infection and rapid expression of viral genes in all cycling, quiescent and postmitotic cells, the use of MLV only allows the infection of the subpopulation of cells that at the time of viral exposure was undergoing mitosis, thus, excluding quiescent and postmitotic ones. Of note, the onset of MLV protein expression will occur in daughters of infected cells.

Thus, to thoroughly characterize the effects of 4D overexpression on adult neurogenesis the inventors generated 4D-expressing HIV and MLV and also designed a system that allows us to conditionally terminate 4D overexpression in infected cells. The inventors generated polycistronic constructs used in the experiments that encode for i) a GFP with a nuclear localization signal (nls), ii) mouse cdk4, and iii) mouse cycling (Fig. 1). Original empty viral backbones were used as described (Wurm et al., 2010) for the HIV lentiviral construct and MLV viruses (Zhao et al., 2006), respectively. cDNA for mouse cdk4 and cyclinD1 (together referred to as 4D) were obtained from the RIKEN consortium (Japan). cdk4 and cyclinD1 are from now on referred to as as 4D. The three genes (GFP^{nls}/cdk4/cyclinD1; from now on referred to as G4D) were linked by the 2A sequences (Fig 1A) 5'-GAGGGCAGAGGAAGTCTTCTAACATGCGGTGACGTGGAGGAGAATCCCGGCCCT-3' (SEQ ID No. 43) or a degenerated code encoding for the same peptide 5'-GAAGGTAGGGGCAGTCTACTTACCTGTGGAGATGTAGAAGAAAACCCAGGACCG-3' (SEQ ID No. 44) that allow the stoichiometric translation of individual proteins from a single mRNA (Tang et al., 2009). Similar constructs were also generated that contain LoxP sites (5'-ATAACTTCGTATAGCATACATTATACGAAGTTAT-3' - SEQ ID No. 45) at the end of the coding region of GFP and cyclinD1 (from now on referred to as G^{Lox}4D^{Lox}) (Fig 1B). By this approach, excision of the 4D cassette upon Cre-mediated recombination could be assessed in individual cells by redistribution of GFP fluorescence from the nucleus to the cytoplasm due to loss of the nls. For control HIV and MLV particles encoding only GFP^{nls} (G), eventually with LoxP sites flanking the nuclear localization signal (nls), were used (Fig 1C).

### Viral preparation and injection.

Viruses were generated and purified according to standard procedures (Tashiro et al., 2006) and injected in the hippocampus of either wildtype C57/B16 or transgenic mice expressing the tamoxifen-dependent Cre recombinase (CreER^{T2}) in NSC. This latter mouse line, the nestinCreERT2/- line (Jackson laboratory, Bar Harbor, Maine, USA Strain B6.Cg(SJL)-TgN(NesCre)1Kln, stock number 003771) was used as described in Imayoshi et al., 2008. Mice were aged between 6 and 11 weeks and infections were performed by using a stereotaxic injection system (coordinates: ±1.6 ML; -1.9 DV; -1.9 AP from dura) (Kopf instruments) following standard surgical procedures (Tashiro et al., 2006). After 1, 2, 3 or 5 weeks, mice were sacrificed and brains collected. Eventually, prior to sacrifice mice were exposed to BrdU (1 mg/mouse injected intraperitoneally (IP) at 12 hours interval for 7 days), tamoxifen (5 mg/mouse IP at 12 hours interval for 2 days), or, finally, tamoxifen followed 3 days later by BrdU..

### Cell fate analysis.

40 µm thick vibratome sections were performed through the entire hippocampus and sections grouped serially every six according to standard stereological methods. Each group, containing 7-8 sections covering the entire hippocampus from its rostral-to-caudal boundary, was used to assess the number of GFP^{nls}+ cells immunoreactive for the markers of S phase and proliferation bromodeoxyuridine (BrdU) or positive for established molecular markers of NSC, NPC or newborn neurons such as Sox2, Tbr2 and DCX, respectively (Kempermann et al., 2004; Zhao et al., 2008). DAPI was used for nuclear staining.

### Results

The results of these experiments are summarized as follows:

### Viral infection allows the coexpression of GFP^{nls} with catalytically active 4D complex:

Infection of HeLa cells with i) GFP^{nls}, or ii) G4D, or G^{Lox}4D^{Lox} HIV or MLV particles followed by assessment of endogenous GFP fluorescence by microscopy revealed the correct localization of GFP^{nls} into the nucleus and its redistribution to the cytoplasm in HeLa cells coexpressing Cre (Fig 6). Moreover, similar experiments followed by western blot analyses for GFP, mouse cdk4, or mouse cyclinD1 showed the efficient co-expression of the three transgenes and the translation of the three individual proteins from a single polycistronic mRNA (Fig 2A and B). Finally, the inventors found that the ectopic 4D complex was catalytically active as revealed by an increased phosphorilation of its downstream target retinoblastoma (Fig 2C and D). Altogether, these experiments showed that the HIV and the MLV here described could be used to temporally control the overexpression of catalytically active 4D complex while being able to distinguish individual 4D overexpressing cells by the expression and localization of GFP.

### Expression of G4D by HIV infection increases proliferation of NSC and NPC:

Histological analyses one week after injection in the adult mouse hippocampus with HIV for GFP^{nls} or G4D revealed that about 10-30% of cells in granular zone expressed GFP and that GFP fluorescence was restricted to the nucleus. Moreover, upon infection with HIV, GFP^{nls} positive cells were evenly distributed in the various layers of the granular zone including neurons, glial cells as well as NSC and NPC (Fig 3A-F).

To investigate possible effects of 4D overexpression on proliferation of NSC or NPC in the hippocampus, the inventors exposed infected mice to repetitive IP injections with the thymidine analogue BrdU, which is irreversibly incorporated in the DNA during S phase and thus allows distinguishing cycling cells from quiescent or postmitotic ones. Infection with control GFP^{nls} viruses showed a proportion of GFP/BrdU+ cells (1-2%) that was in line with the value reported in the literature (Fig 3B and C; white symbols) (Mao et al., 2009). However, the inventors observed that already 1 week after 4D overexpression the proportion of GFP/BrdU+ cells had increased by ca. 70% in G4D as compared to with GFP^{nls} viral infection (Fig 3C). Importantly, while in control conditions the proportion of BrdU+ cells remained overall constant overtime, the increase in the proportion of BrdU+ cells after 4D overexpression become more prominent at longer survival times reaching, at the third week, an increase by over 100% as compared to control (Fig 3C).

Importantly, the increased proportion of BrdU+ cells in the hippocampus upon 4D overexpression can be explained by fourth different possibilities, or a combination thereof. Specifically, first, 4D overexpression could induce acceleration of the cell cycle with no effect neither on quiescence nor differentiation as, in fact, a shorter G1 would induce cycling cells to enter S phase more often leading to an increase in the proportion of BrdU+ cells. Alternatively, second, 4D overexpression could inhibit the differentiation of NSC and/or NPC inducing them to generate more stem/progenitor cells instead of postmitotic neurons, thus increasing the proportion of BrdU+ cells. Moreover, third, 4D overexpression could induce quiescent NSC to enter the cell cycle and thus divide, which also would increase the proportion of BrdU+ cells. Finally, fourth, 4D overexpression may be toxic in postmitotic cells thereby triggering apoptosis in neurons leading to an increased proportion of cycling cells through their increased survival. The inventors aimed at discriminating between these possibilities by a series of experiments.

### Expression of G4D by HIV infection increases the proportion of NSC and NPC:

The inventors first assessed the proportion of NSC or NPC within the population of GFP^{nls}+ cells 3 weeks after infection with either GFP^{nls} or G4D lentiviruses i.e.: when effects on proliferation were stronger. Immunohistochemistry for the molecular markers Sox2 and Tbr2 were used to identify NSC and NPC, respectively (Kempermann et al., 2004; Zhao et al., 2008) (Fig 3D and G).

Infection with control, GFP^{nls} HIV showed a proportion of Sox2+ (ca. 1.8%) and Tbr2+ (0.8%) cells that was consistent with what reported in the literature (Suh et al., 2007; Hodge et al., 2008) indicating that infection *per se* does not alter the proportion of NSC or NPC. In contrast, the inventors found that upon G4D infection the proportion of Sox2+ and Tbr2+ cells had increased by ca. 140% and 80%, respectively (Fig 3E and F). This finding excludes the possibility that the primary effect of 4D overexpression is solely to change the length of the G 1 phase of the cell cycle of NPC without influencing quiescence of NSC or differentiation of either kind of precursor. In fact, a shorter G1, without a change in neither quiescence nor differentiation, while still changing the rate of BrdU+ incorporation cannot lead to a change in the abundance of different cell types. The inventors thus conclude that the primary effect of 4D overexpression is to induce exit from quiescence of NSC and inhibit differentiation.

### Expression of G4D by HIV infection reduces the proportion of newborn neurons:

To validate the conclusion that a possible effect of 4D overexpression is to inhibit the differentiation of NSC and/or NPC, the inventors assessed the proportion of newborn neurons that were generated during the three weeks interval from the infection with either GFP^{nls} or G4D viruses. To identify newborn neurons, the inventors performed immunohistochemistry for doublecortin (DCX), a marker being transitorily expressed for two weeks after neuronal birth (Kempermann et al., 2004; Zhao et al., 2008).

Particularly instructive for this analysis were sections in which GFP^{nls}+ cells after G4D infection were more concentrated, or occasionally even limited, to a given region of the hippocampus such as its most dorsal or ventral portion. In essence, this allowed the comparison, within the same section, of the abundance of DCX+ cells in the 4D-targeted and internal negative control part of the hippocampus. The inventors consistently found an evident reduction of DCX+ cells in the G4D-targeted region of the hippocampus (Fig 4G), which was independent on whether this portion was dorsal or ventral.

In essence, 4D overexpression leads to a reduction of newborn neurons, which is consistent with the increase in NSC and NPC previously described and corroborated the hypotesis that one of the effects of 4D overexpression is to inhibit differentiation and promote proliferation.

### Expression of G4D by MLV has no effect on newborn neurons:

The previous experiments show that the increased proliferation is caused by a decreased differentiation of NSC and NPC. However, it still remains unclear whether quiescence of NSC is at all influenced by this manipulation. More importantly, it should be noticed that the increased proportion of Sox2+ and Tbr2+ cells and decreased proportion DCX+ neurons could simply be caused by neuronal death after 4D overexpression. In fact, the 4D complex is considered to be a potent positive regulator of cell cycle progression and it cannot be excluded that overexpression of 4D in postmitotic neurons may lead to cell stress and apoptosis. In other words, all phenotypes described so far could simply be explained by death of neurons overexpressing 4D.

To exclude this possibility, the inventors performed similar manipulations using G4D MLV particles that, in contrast to HIV, can only integrate their genome upon mitosis. Since in normal conditions the vast majority of NSC are quiescent, only mitotic NPC would be targeted. As a result, daughters of the infected, dividing NPC, i.e. mostly newborn neurons, will start to overexpress the 4D cassette, which they inherited from their mother. That is, by this approach the inventors can investigate the effects of 4D overexpression in the subpopulation of newborn neurons generated soon after infection and in the residual population of NPC that did not generate neurons but generated more NPC instead. Importantly, the use of MLV would not influence NSC due to their quiescent state.

MLV were injected in the hippocampus and effects on BrdU, DCX and cell morphology investigated 4 days, 1 or 2 weeks after infection with either GFP^{nls} or G4D MLV (Fig 5). In no case could the inventors see any change between control and 4D viruses in any of the parameters analyzed, which always returned proportions consistent with the ones reported in the literature in control conditions (Fig 5B) (Jagasia et al., 2009; Esposito et al., 2005).

In essence, overexpression of 4D in newborn neurons does not impair their survival and targeting of NPC does not change their commitment to generate neurons. Considering that i) most of the increase in the proportion of BrdU+ cells was observed to occur already during the first week after infection (Fig 3C) and ii) that the increase in the proportion of NSC was more prominent than that observed in NPC (Fig 3E versus 3F), the inventors conclude that the primary effect of 4D overexpression is to induce quiescent NSC to enter the cell cycle and generate more NSC and, thus, to expand.

### Infection with G^{Lox}4D^{Lox} lentiviruses allows to temporally control the expansion of NSC:

Having shown that an acute and tissue-specific 4D overexpression in the adult mouse hippocampus triggers the expansion of NSC, the fundamental question remains as to whether or not this effect can be temporally restricted in order to increase the neuronal output in the brain. In fact, the main hope for using somatic stem cells in therapy is not only that their expansion could be increased but also that their differentiation could be temporally controlled. As to now, the inventors have shown that 4D overexpression increases the proportion of NSC. Thus, the inventors reasoned, if the inventors should be able to stop the expression of the ectopic 4D complex by, for example, removing the 4D cassette from the genome of infected cells, NSC would now be able to undergo physiological differentiation. To investigate this, the inventors generated lentiviral constructs containing LoxP sites flanking the last codon of GFP and cyclinD1 such as to excise the nls of GFP and the whole 4D cassette upon Cre-mediated recombination (see Fig 1B and C for a map of the G^{Lox}4D^{Lox} and control constructs, respectively) that revealed to be effective in HeLa cells (Fig 6A). Injections were performed in *nestin*^{CreERT2/-} mice, in which tamoxifen-dependent Cre-recombination was shown to occur specifically in NSC and NPC (Imayoshi et al., 2008). Three weeks after infection, mice were administered tamoxifen or vehicle for four days. Brains were collected ten days later for analyses, which revealed redistribution of GFP fluorescence from the nucleus to the cytoplasm of putative NSC (Fig 6B; compare with Fig 3A).

Altogether these data show that the inventors can acutely and tissue-specifically overexpress the 4D complex in the adult neurogenic niche while maintaining the ability to conditionally stop this effect. In essence, the inventors developed as system that allows to temporally control the expansion of NSC and their switch to differentiation *in vivo.*

### Example 2: Expansion of NSC and NPC in vitro:

The lentiviral vector as described in Example 1 can also be used to expand NSC or NPC *in vitro*. However, for *in vitro* applications, not only viral particles but also any other transfection system with plasmids or DNA/RNA fragments or delivery of proteins can be used, including the use of drugs that pharmacologically act to promote cdk/cyclin expression or their catalytic activity. For the expansion of NPC a MLV as described in example 1 is preferred.

### Example 3: construct for the viral infection of human NSC (or NPC) in vivo:

For the infection of NSC (or NPC) in the human brain the fluorescent reporter GFP is replaced directly by CreER^{T2} to mediate the complete removal of the exogenous DNA from human cell at the desired time by administration of tamoxifen. An example of a vector for use in humans is shown in Fig 1D (without GFP insertion), where, importantly, expression of CreER^{T2} will lead to the complete removal of the entire vector upon tamoxifen administration thereby removing any exogenous DNA from the genome of the patient. In essence the vector is designer as to allow its efficient removal at any time point after infection.

The CreER^{T2}/GFP/cdk/cyclin vector shown in Fig. 1D with GFP insertion is preferred for the expansion of NSC (or NPC) *in vitro* for cell transplantation approaches. For NSC the lentivirus and for NPC the MLV is preffered.

### Example 4: Generation of transgenic mice for the inducible expansion of neural stem cells:

Another direct application of the invention is the generation of transgenic mice for the inducible expansion of stem cells. This can be achieved by combining the many technologies available for inducible control of gene expression *in vivo,* some of which described above, as a means to directly influence the fate of all cells of a given tissue and not only the subpopulation that is being targeted targeted by viral infection or the other methods described above.

For example, using the Doxycycline-inducible system the inventors have generated tissue-specific, tetracycline-inducible (Tet-On) cdk/cyclin mouse lines for the temporal and spatial manipulation of cdk/cyclin expression in NSC thus controlling their proliferation vs. differentiation during embryonic development or adulthood. Specifically transgenic mouse lines were created in which a bidirectional Tet-promoter drives simultaneous expression of, in one direction, a cyclin and a GFP and, in the other direction, a cdk and a luciferase, which expression can be used for precise quantification of responsiveness upon Doxycycline administration (Fig 1E). Clearly, the GFP and/or the luciferase can be replaced by any other desired gene for cell selection, identification or genetic recombination as described above. Importantly, these Tet-On responder mouse lines were crossed with several other transgenic mouse lines in which Tet expression in under the control of cell-specific promoters. These bigenic mice (and any other additional crossing of the responder with any Tet expressing line), allows to overcome the only limitation of the vector trasnduction systems described until now, which, as efficient as they can be, only allow transduction in a in subpopulation (relatively small) of cells rather than in all the relevant cells of a given tissue.

If applied to NSC, this system will trigger much greater effects on expansion that can be applied for a multitude of different purposes and contexts e.g.: during i) embryonic development, ii) adulthood, or iii) in mouse model of neurodegenerative diseases or CNS injuries.

For instance, expansion of NSC during adulthood may allow to address the hitherto elusive role of adult neurogenesis for elaborate function such as memory and learning. Expansion of NSC in models of CNS disease or injury can be use to establish platforms for the investigation of novel approaches of regenerative therapies in animal models.

Finally, the responder Dox-inducible cdk/cyclin mouse lines generated by the inventors can be crossed with any of the many available tissue-specific Tet-On activator lines thus allowing the expansion/differentiation of any somatic stem cell in vivo by simple addition/removal of Doxycycline.

METHOD: Using RedET recombination (Vintersten et al., 2008), the inventors generated bacterial artificial chromosomes (BAC) covering the genomic locus of NSC/NPC cell markers, such as GFAP and Sox2, in which the endogenous gene was replaced with a rtTA cassette. These modified BACs were then used to generate transactivator transgenic mouse lines by male pronuclear injection (Vintersten et al., 2008). In addition, vectors were generated in which a bidirectional Tet-Op promoter drives the expression of a G 1-specific cyclin, its cdk, and two reportes, such as RFP and luciferase which are linked by 2A peptides (a similar construct with GFP is shown in Fig 1E). These vectors were also used to generate responder transgenic lines by male pronuclear injection.

RESULTS: Of the many transactivator lines generated we found, in particular for Sox2-rtTA, some faithfully reproducing the Sox2 pattern and tissue-specificity for rtTA expression. This was confirmed by in situ hybrydization of embryonic and adult brain tissue. Similarly, various cdk/cyclin responder lines were screened by transfecting primary fibroblast cultures obtained their from for luciferase assay and RFP expression upon rtTA transduction and administration of doxycycline. Western blott analysis for cdk and cyclins, similarly to the experiments described above and in Fig 2 upon viral infection, corroborated the correct expression of the transgenes upon rtTA expression and doxycycline administration. Finally, double transactivator/responder bigenic lines were obtained and overexpression of the transgenes confirmed by detection of RFP *in vivo.* The responder lines are crossed with other available tissue-specific transactivator lines to allow inducible and reversible overexpression of cdk/cyclins in virtually any tissue in vivo. This system is also combined with Cre-recombination by crossing the responder mice (Tet-On^{cdk/cyclin}) with a transgenic line in which rtTA expression in activated after Cre-mediated removal of a stop cassette (^{lox}stop^{lox}-rtTA). The crossing of the bigenic Tet-On^{cdk/cycyclin}/ ^{lox}stop^{lox}-rtTA with any tissue-specific Cre-expressing system further extend the possibily to study any tissue of interest using the already available mouse lines expressing Cre.

### ABBREVIATIONS

The following abbreviations are used in the description:
- **CNS**: central nervous system,
- **NSC**: neural stem cell,
- **NPC**: neural progenitor cell,
- **Cdk**: cyclin dependent kinase,
- **4D**: cdk4/cyclinD1 complex,
- **GFP**: green fluorescent protein,
- **Nls**: nuclear localization signal,
- **G4D**: polycistronic construct encoding for GFP^{nls}, cdk4 and cyclinD1,
- **G^{Lox}4D^{Lox}**: G4D construct containing LoxP sites at the 3' end GFP, prior to the nls, and cyclinD1,
- **CreER^{T2}**: tamoxifen-dependent Cre recombinase,
- **IP**: intraperitoneal,
- **BrdU**: bromodeoxyuridine,
- **HIV**: human immunodeficiency virus (or vector derived from),
- **MLV**: Moloney murine leukemia virus (or vector derived from).

### REFERENCES

The following non-patent literature is cited in the description:
Anastassiadis K et al. 2009. Dis Model Mech 2:508-515.
Barnabé-Heider F et al. 2008. Nat Methods 5:189-196.
Bordignon C, Roncarolo MG 2002. Nat Immunol 3:318-321.
Buchholz F 2009. Curr Opin Biotechnol 20:383-389.
Calegari F, Huttner WB 2003. J Cell Sci 116:4947-4955.
Cameron HA, McKay RD 2001. J Comp Neurol 435:406-417.
Cheng T et al. 2000. Science 287:1804-1808.
Chenn A, Walsh CA 2002. Science 297:365-369
De Felipe P et al. 2005 Trends Biotechnol 24:68-75
Deiters A 2009. Curr Opin Chem Biol 13:678-686.
Espósito MS et al. 2005. J Neurosci 25:10074-86.
Fuchs E 2009. Cell 137:811-819.
Hodge RD et al. 2008. J Neurosci 28:3707-17.
Imayoshi I et al. 2008. Nat Neurosci 11:1153-1161.
Jagasia R et al. 2009. J Neurosci 29:7966-77.
Kageyama R et al. 2008 Nat. Neurosci. 11:1247-1251.
Kempermann G et al. 2004. Trends Neurosci 27:447-452.
Kippin TE et al. 2005. Genes Dev 19:756-767.
Kriegstein A, Alvarez-Buylla A 2009. Annu Rev Neurosci 32:149-184.
Lange C et al. 2009. Cell Stem Cell 5:320-331.
Langel U 2006. Handbook of Cell-Penetrating Peptides 5-28 (CRC -Taylor & Francis Group).
Li L, Clevers H 2010. Science 327:542-545.
Lindvall O, Kokaia Z 2006. Nature 441:1094-1096.
Lowenstein PR, Castro MG 2002. Curr Opin Mol Ther 4:359-371.
Lukaszewicz A et al 2002. J Neurosci 22:6610-6622
Mao Y et al. 2009. Cell 136:1017-31.
Okano H, Temple S 2009. Curr Opin Neurobiol 19:112-119.
Okano H et al. 2007. J Neurochem 102:1459-1465.
Orford KW, Scadden DT 2008. Nat Rev Genet 9:115-128.
Orkin SH, Zon LI 2008. Cell 132:631-644.
Prall OW et al. 1997. J Biol Chem. 272:10882-94.
Reynolds BA, Vescovi AL 2009. Cell Stem Cell 5:466-467; author reply 468-469.
Roe T, Reynolds TC, Yu G, Brown PO 1993. EMBO J 12:2099-108.
Salomoni P, Calegari F 2010. Trends Cell Biol doi:l0.1016/j.tcb.2010.01.006.
Singh AM, Dalton S 2009. Cell Stem Cell 5:141-149.
Smith A 2006. Nature 441:1060.
Suh H et al.. 2009. Cell Stem Cell. 1:515-28.
Tang W et al. 2009. J Neurosci 29:8621-8629.
Tashiro A et al. 2006. Nat Protoc 1:3049-3055.
Taupin P et al. 2000. Neuron 28:385-397.
Vescovi AL, Snyder EY 1999. Brain Pathol 9:569-598.
Vintersten K et al. 2008 Methods Mol Biol. 415:83-100.
Woolard K, Fine HA 2009. Cell Stem Cell 4:466-467.
Wurm M at al. 2010 J Gene Med 12:137-146.
Zhao C et al. 2006 J Neurosci 26:3-11.
Zhao C et al. 2008. Cell 132:645-660.
Zhou H et al. 2009. Cell Stem Cell 4:381-384.

## Claims

1. Use of a vector that contains genes encoding for cyclin-dependent kinase 4 and cyclin D1, wherein the genes encoding cyclin-dependent kinase 4 and cyclin D1 are separated by a 2A peptide encoding nucleic acid sequence, for the *in vitro* expansion of adult somatic stem cells by transfection or transduction of said adult somatic stem cells with said vector.

2. Use according to claim 1, **characterized in that** the adult somatic stem cells are selected from neural stem cells, hematopoietic stem cells, mesenchymal stem cells, endothelial stem cells and tissue-specific stem cells of the skin, gut, muscles or hair follicles.

3. Use according to claim 1, **characterized in that** the adult somatic stem cells are neural stem cells.

4. Use according to one of the preceding claims, **characterized in that** the adult somatic stem cells are quiescent.

5. Use according to one of the claims 1 to 4, **characterized in that** the vector comprises a fusion gene wherein the genes encoding cyclin-dependent kinase 4 and cyclin D1 result in an equimolar overexpression of cyclin-dependent kinase 4 and cyclin D1.

6. Use according to one of the claims 1 to 5, **characterized in that** the vector comprises a construct that allows an inducible expression of the genes encoding cyclin-dependent kinase 4 and cyclin D1.

7. Use according to one of the claims 1 to 6, **characterized in that** the vector additionally contains a gene encoding a reporter protein and/or a nucleotide sequence encoding an epitope tag.

8. Adult somatic stem cell transfected or transduced by a vector as defined in one of the claims 1 to 7.

9. Non human animal transfected or transduced by a vector as defined in one of the claims 1 to 7 or comprising adult somatic stem cells according claim 8.

10. Use according to claim 1 to 7, adult somatic stem cell according to claim 8 or non human animal according to claim 9 in life science, neuroscience and/or pharmaceutical research.

11. *In vitro* method for the expansion of adult somatic stem cells with the steps:
a.) providing a vector as defined in one of the claims 1 to 7,
b.) introducing said vector into adult somatic stem cells by transfection or transduction,
c.) overexpression of cyclin-dependent kinase 4 and cyclin D1 in the cells transfected or transduced in step b.), resulting in proliferation of the somatic stem cells transfected or transduced in step b.).

12. Method according to claim 11 using a vector as defined in claim 6 with the additional step of inducing the knockout of the cyclin-dependent kinase 4 and the cyclin D 1 genes inserted into the adult somatic stem cells after step b.) to stop the induced proliferation of the transfected or transduced adult somatic stem cells and to induce differentiation of the adult somatic stem cells.

13. Method according to claim 11 or 12, **characterized in that** the adult somatic stem cells are quiescent.

14. Vector as defined in one of the claims 1 to 7 for use in the expansion of adult neural stem cells for the treatment of a human or an animal having a disease, disorder or condition of the central nervous system.

## Patentansprüche

1. Verwendung eines Vektors, der Gene enthält, die für cyclinabhängige Kinase 4 und Cyclin D1 kodieren, wobei die für cyclinabhängige Kinase 4 und Cyclin D1 kodierenden Gene durch ein 2A Peptid voneinander getrennt sind, für die *in vitro* Expansion adulter somatischer Stammzellen durch Transfektion oder Transduktion besagter adulter somatischer Stammzellen mit besagtem Vektor.

2. Verwendung gemäß Anspruch 1, **dadurch gekennzeichnet, dass** die adulten somatischen Stammzellen ausgewählt sind aus neuralen Stammzellen, hämatopoietischen Stammzellen, mesenchymalen Stammzellen, Endothelstammzellen und gewebespezifischen Stammzellen von Haut, Darm, Muskel oder Haarfollikeln.

3. Verwendung gemäß Anspruch 1, **dadurch gekennzeichnet, dass** die adulten somatischen Stammzellen neurale Stammzellen sind.

4. Verwendung gemäß einem der vorhergehenden Ansprüche, **dadurch gekennzeichnet, dass** die adulten somatischen Stammzellen ruhende Stammzellen sind.

5. Verwendung gemäß einem der Anspruche 1 bis 4, **dadurch gekennzeichnet, dass** der Vektor ein Fusionsgen enthält, bei dem die für cyclinabhängige Kinase 4 und Cyclin D1 kodierenden Gene in einer äquimolaren Überexpression von cyclinabhängiger Kinase 4 und Cyclin D1 resultieren.

6. Verwendung gemäß einem der Ansprüche 1 bis 5, **dadurch gekennzeichnet, dass** der Vektor ein Konstrukt umfasst, welches die induzierbare Expression der für cyclinabhängige Kinase 4 und Cyclin D1 kodierenden Gene erlaubt.

7. Verwendung gemäß einem der Ansprüche 1 bis 6, **dadurch gekennzeichnet, dass** der Vektor zusätzlich ein Gen enthält, das für ein Reporterprotein kodiert und/oder eine Nukleotidsequenze, die für ein Epitoptag kodiert.

8. Adulte somatische Stammzelle transfiziert oder transduziert mit einem Vektor, wie in einem der Ansprüche 1 bis 7 definiert.

9. Nicht-menschliches Tier transfiziert oder transduziert mit einem Vektor, wie in einem der Ansprüche 1 bis 7 definiert oder umfassend adulte somatische Stammzellen gemäß Anspruch 8.

10. Verwendung gemäß Anspruch 1 bis 7, adulte somatische Stammzelle gemäß Anspruch 8 oder nicht-menschliches Tier gemäß Anspruch 9 in den Biowissenschaften, Neurowissenschaften und/oder der pharmazeutischen Forschung.

11. *In vitro* Verfahren zur Expansion adulter somatischer Stammzellen mit den Schritten:
a.) Bereitstellung eines Vektors, wie in einem der Ansprüche 1 bis 7 definiert,
b.) Einbringen besagten Vektors in adulte somatische Stammzellen durch Transfektion oder Transduktion,
c.) Überexpression von cyclinabhängiger Kinase 4 und Cyclin D1 in den in Schritt b.) transfizierten oder transduzierten Zellen, resultierend in der Proliferation der in Schritt b.) transfizierten oder transduzierten somatischen Stammzellen.

12. Verfahren gemäß Anspruch 11 unter Verwendung eines Vektors, wie in Anspruch 6 definiert, mit dem nach Schritt b.) durchgeführten zusätzlichen Schritt der Einbringung eines Knockouts der Gene für cyclinabhängige Kinase 4 und Cyclin D1, die in die adulten somatischen Stammzellen eingebracht wurden, um die induzierte Proliferation der transfizierten oder transduzierten adulten somatischen Stammzellen zu stoppen und die Differenzierung der adulten somatischen Stammzellen zu induzieren.

13. Verfahren gemäß Anspruch 11 oder 12, **dadurch gekennzeichnet, dass** die adulten somatischen Stammzellen ruhende Stammzellen sind.

14. Vektor, wie in einem der Ansprüche 1 bis 7 definiert zur Verwendung bei der Expansion adulter neuraler Stammzellen zur Behandlung eines Menschen oder Tiers mit einer Erkrankung, Funktionsstörung oder Beschwerde des zentralen Nervensystems.

## Revendications

1. Utilisation d'un vecteur qui contient des gènes codants pour une kinase dépendant d'une cycline 4 et une cycline D1, dans laquelle les gènes codant une kinase dépendant d'une cycline 4 et une cycline D1 sont séparés d'un peptide 2A codant une séquence d'acides nucléiques en vue d'une expansion in vitro de cellules souches somatiques adultes par transfection ou transduction desdites cellules souches somatiques adultes à l'aide dudit vecteur.

2. Utilisation selon la revendication 1, **caractérisée en ce que** les cellules souches somatiques adultes sont sélectionnées à partir de cellules souches neuronales, de cellules souches hématopoïétiques, de cellules souches mésenchymateuses, de cellules souches endothéliales et de cellules souches spécifiques à un tissu de la peau, de l'intestin, des muscles ou des follicules pileux.

3. Utilisation selon la revendication 1, **caractérisée en ce que** les cellules souches somatiques adultes sont des cellules souches neuronales.

4. Utilisation selon l'une des revendications précédentes, **caractérisée en ce que** les cellules souches somatiques adultes sont quiescentes.

5. Utilisation selon l'une des revendications 1 à 4, **caractérisée en ce que** le vecteur comprend un gène de fusion où les gènes codant une kinase dépendant d'une cycline 4 et une cycline D1 résultent en une surexpression équimolaire de kinase dépendant d'une cycline 4 et de cycline D1.

6. Utilisation selon l'une des revendications 1 à 5, **caractérisée en ce que** le vecteur comprend un élément de construction qui permet une expression inductible des gènes codant une kinase dépendant d'une cycline 4 et une cycline D1.

7. Utilisation selon l'une des revendications 1 à 6, **caractérisée en ce que** le vecteur contient de plus un gène codant une protéine rapporteur et/ou une séquence de nucléotides codant une étiquette épitope.

8. Cellule souche somatique adulte transfectée ou transduite par un vecteur selon l'une des revendications 1 à 7.

9. Animal non humain transfecté ou transduit par un vecteur selon l'une des revendications 1 à 7 ou comprenant des cellules souches somatiques adultes selon la revendication 8.

10. Utilisation selon les revendications 1 à 7, cellule souche somatique adulte selon la revendication 8, ou animal non humain selon la revendication 9 en sciences de la vie, en neuroscience et/ou en recherche pharmaceutique.

11. Procédé in vitro d'expansion de cellules souches somatiques adultes ayant les étapes :
a.) de fourniture d'un vecteur selon l'une des revendications 1 à 7,
b.) d'introduction dudit vecteur dans des cellules souches somatiques adultes par transfection ou transduction,
c.) de surexpression de kinase dépendant d'une cycline 4 et d'une cycline D1 dans les cellules transfectées ou transduites lors de l'étape b.), résultant en une prolifération des cellules souches somatiques transfectées ou transduites lors de l'étape b.).

12. Procédé selon la revendication 11, utilisant un vecteur selon la revendication 6, ayant l'étape supplémentaire d'induction de l'inactivation des gènes de la kinase dépendant d'une cycline 4 et de la cycline D1 insérés dans les cellules souches somatiques adultes après l'étape b.) afin de stopper la prolifération induite des cellules souches somatiques adultes transfectées ou transduites et afin d'induire une différenciation des cellules souches somatiques adultes.

13. Procédé selon la revendication 11 ou 12, **caractérisé en ce que** les cellules souches somatiques adultes sont quiescentes.

14. Vecteur selon l'une des revendications 1 à 7, destiné à une utilisation dans l'expansion des cellules souches neuronales adultes pour le traitement d'un humain ou d'un animal présentant une maladie, un trouble ou un état du système nerveux central.
